# EUROPEAN PATENT SPECIFICATION

(11) **EP 1 253 881 B1**
(45) Date of publication and mention of the grant of the patent: **14.09.2005**
(21) Application number: 01902952.9
(22) Date of filing: 08.02.2001
(51) Int. Cl.: A61F 2/68, A61F 5/44, A61F 2/00

(54) **ANAL INCONTINENCE TREATMENT WITH CONTROLLED WIRELESS ENERGY SUPPLY**
BEHANDLUNG ANALER INKONTINENZ MIT KONTROLLIERTER DRAHTLOSER ENERGIEVERSORGUNG
TRAITEMENT DE L'INCONTINENCE ANALE AVEC UNE ALIMENTATION EN ENERGIE COMMANDEE SANS FIL

(30) Priority: 10.02.2000 US 502089; 10.02.2000 US 502091
(43) Date of publication of application: 06.11.2002
(60) Divisional application: 05015424.4
(73) Proprietor: Potencia Medical AG, 6341 Baar (CH)
(72) Inventor: FORSELL, Peter, CH-6313 Menzingen (CH); JAKOBSSON, Arne, F-06160 Juan Les Pins (FR)
(74) Representative: Berglund, Stefan
(86) International application number: PCT/SE2001/000254
(87) International publication number: WO 2001/058390

(56) References cited:
- FR-A1- 2 692 777
- US-A- 5 509 888

## Description

The present invention relates to an anal incontinence treatment apparatus, comprising a restriction device implantable in a patient suffering from anal incontinence for engaging the colon or rectum to form a restricted faecal passageway in the colon or rectum, wherein the restriction device is operable to change the restriction of the faecal passageway. The term "patient" includes an animal or a human being.

Anal incontinence is a wide-spread disease. Several kinds of sphincter plastic surgery are used today to remedy anal incontinence. There is a prior manually operated sphincter system in an initial clinical trial phase where a hydraulic sphincter system connected to an elastic reservoir (balloon) placed in the scrotum is developed. A disadvantage of this system is that thick, hard fibrosis is created around the reservoir by pump movements making the system useless sooner or later.

U.S. Pat. No. 5,593,443 discloses a hydraulic anal sphincter under both reflex and voluntary control. A pressure controlled inflatable artificial sphincter is disclosed in U.S. Pat. No. 4,222,377.

U.S. Pat. No. 5,509,888 describes another implantable valve device by means of which a flow of fluid can be constrictively occluded in a passageway, such as the colon.

The object of the present invention is to provide a new convenient anal incontinence treatment apparatus, the performance of which may be affected by the patient at any time after operation, in particular when need arise over the course of a day, so that the patient substantially always is satisfied or comfortable.

This object is achieved by an anal incontinence treatment apparatus described in claim 1, which is characterised in that an external source of energy is provided, which is intended to be external to the patient's body when the restriction device is implanted therein. The external source of energy is adapted to release wireless energy. An implantable energy transforming device is also provided for transforming the released wireless energy into storable energy. Furthermore, the internal source of energy is capable of storing the storable energy, and the control device is adapted to control the internal source of energy to supply energy for the operation of the restriction device. Additionally, the control device, in turn, comprises a first control unit and a second control unit. The first control unit is operable from outside the patient's body for providing a control signal, and the second control unit implantable in the patient and adapted to control the restriction device in response to the control signal.

As a result, the advantage is achieved that the restriction device can be non-invasively operated, when the restriction device has to be adjusted. Furthermore, the apparatus of the invention provides a simple and effective control of the energy supplied to implanted components of the apparatus which ensures an extended and reliable functionality of the apparatus, possibly for the rest of the patient's life and at least many years.

The control device may also control the restriction device. The control device may comprise an internal control unit, preferably including a microprocessor, implantable in the patient for controlling the restriction device. The control device may further comprise an external control unit outside the patient's body, wherein the internal control unit is programmable by the external control unit, for example for controlling the restriction device over time. Alternatively, the internal control unit may control the restriction device over time in accordance with an activity schedule program, which may be adapted to the patient's needs.

A great advantage is that the patient is enabled to adjust the restriction of the faecal passageway by using the control device whenever he likes during the day.

Conveniently, the external control unit may load the internal control unit with data in accordance with a loading mode only authorized for a doctor. For specialized controls of the restriction device, the external control unit may control the internal control unit in accordance with a doctor mode only authorized for the doctor. For simple controls of the restriction device, the external control unit may control the internal

A great advantage is that the patient is enabled to adjust the restriction of the faecal passageway by using the control device whenever he likes during the day.

Conveniently, the external control unit may load the internal control unit with data in accordance with a loading mode only authorized for a doctor. For specialized controls of the restriction device, the external control unit may control the internal control unit in accordance with a doctor mode only authorized for the doctor. For simple controls of the restriction device, the external control unit may control the internal control unit in accordance with a patient mode permitted for the patient. Thus, by using the external control unit in accordance with different modes it is possible to have certains functions of the restriction device controlled by the patient and other more advanced functions controlled by the doctor, which enables a flexible post-operation treatment of the patient.

The control device may be adapted to control the external source of energy to intermittently release energy in the form of a train of energy pulses, for direct use in connection with the operation of the restriction device. In accordance with a suitable embodiment the control device controls the internal source of energy to release electric energy, and the apparatus further comprises an implantable capacitor for producing the train of energy pulses from the released energy. In this case the term *"direct"* is used to mean, on one hand, that the released energy is used while it is being released by the control device, on the other hand, that the released energy may be somewhat delayed, in the order of seconds, by for instance an energy stabiliser before being used in connection with the operation of the restriction device. The restriction device may be operable in non-manual, a non-magnetic or non-mechanical manner by use of the released energy.

In accordance with a preferred embodiment of the invention, the apparatus comprises implantable electrical components including at least one, or only one single voltage level guard and a capacitor or accumulator, wherein the charge and discharge of the capacitor or accumulator is controlled by use of the voltage level guard. As a result, there is no need for any implanted current detector and/or charge level detector for the control of the capacitor, which makes the apparatus simple and reliable.

Generally, the apparatus further comprises an operation device implantable in the patient for operating the restriction device, wherein the control device controls the operation device to operate the restriction device. The control device may directly power the operation device with energy released from the external source of energy and/or power other implantable energy consuming components of the apparatus. In this case the term *"directly"* is used to mean, on one hand, that the operation device is powered with released energy while the latter is being released by the control device, on the other hand, that the released energy may be somewhat delayed, in the order of seconds, by for instance an energy stabiliser before powering the operation device. The advantage of directly using energy as it is released is that the apparatus can be of a very simple design and the few components involved makes the apparatus reliable.

The restriction device may be non-inflatable, i.e. with no hydraulic fluid involved for the adjustments of the restriction device. This eliminates problems with fluid leaking from the restriction device.

The operation device may comprise hydraulic means and at least one valve for controlling a fluid flow in the hydraulic means. The control device may suitably comprise a wireless remote control for controlling the valve. The restriction device may comprise hydraulic means and the operation device may comprise a reservoir forming a fluid chamber with a variable volume connected to the hydraulic means. The operation device may distribute fluid from the chamber to the hydraulic means by reduction of the volume of the chamber and withdraw fluid from the hydraulic means to the chamber by expansion of the volume of the chamber.

The external source of energy may be of any conceivable kind, such as a nuclear source of energy or a chemical source of energy.

The internal source of energy preferably comprises an electric accumulator. The electric accumulator may comprise at least one capacitor or at least one rechargeable battery, or a combination of at least one capacitor and at least one rechargeable battery. A battery may be implantable in the patient for supplying electric energy to implanted electric energy consuming components of the apparatus, in addition to the supply of wireless energy. Where the control device comprises an implantable control unit the electronic circuit thereof and the restriction device may be directly powered with transformed wireless energy, or energy from either the implantable internal source of energy or battery.

The wireless energy may be directly used for operation of the restriction device, i.e. the restriction device is operated as the wireless energy is released from the external source of energy by the control device. In this case the term *"directly"* is used to mean, on one hand, that the restriction device is promptly operated by using the released energy whithout first storing the latter, on the other hand, that the released energy may be somewhat delayed, in the order of seconds, by for instance an energy stabiliser before being used for the operation of the restriction device. As a result, a very simple control of the restriction device is achieved and there are only a few implanted components of the apparatus. For example, there is no implanted complicated signal control system. This gives the advantage that the apparatus will be extremely reliable.

Generally, the control device controls and directly or indirectly powers the operation device with wireless energy released from the external source of energy and/or powers other implanted energy consuming components of the apparatus.

In a first particular embodiment of the invention, the operation device comprises a motor, preferably an electric motor which may have electrically conductive parts made of plastics. The motor may include a rotary motor, wherein the control device is adapted to control the rotary motor to rotate a desired number of revolutions. Alternatively, the motor may include a linear motor, or a hydraulic or pneumatic fluid motor, wherein the control device is adapted to control the fluid flow through the fluid motor. Motors currently available on the market are getting smaller and smaller. Furthermore, there is a great variety of control methods and miniaturized control equipment available. For example, a number of revolutions of a rotary motor may be analyzed by a Hall-element just a few mm in size.

In a second particular embodiment of the invention, the control device is adapted to shift polarity of the released energy to reverse the operation device. The operation device may suitably comprise an electric motor and the released energy may comprise electric energy.

In a third particular embodiment of the invention, the restriction device is operable to perform a reversible function and there is a reversing device implantable in the patient for reversing the function performed by the restriction device. Such a reversing function preferably involves enlarging and restricting the faecal passageway by the restriction device, suitably in a stepless manner. In this connection, the control device suitably controls the reversing device, which may include a switch, to reverse the function performed by the restriction device. The reversing device may comprise hydraulic means including a valve for shifting the flow direction of a fluid in the hydraulic means. Alternatively, the reversing device may comprise a mechanical reversing device, such as a switch or a gearbox.

Where the reversing device comprises a switch the control device suitably controls the operation of the switch by shifting polarity of released energy supplied to the switch. The switch may comprise an electric switch and the source of energy may supply electric energy for the operation of the switch. The switch mentioned above may comprise an electronic switch or, where applicable, a mechanical switch.

In accordance with the third particular embodiment, the operation device preferably comprises a motor, wherein the reversing device reverses the motor.

In a fourth particular embodiment of the invention, the restriction device comprises hydraulic means, for example including an expansible/contractible cavity for fluid. Preferably, the operation device is adapted to conduct hydraulic fluid in the hydraulic means, and comprises a motor, a valveless fluid conduit connected to the hydraulic means of the restriction device, and a reservoir for fluid, wherein the reservoir forms part of the conduit. The operation device suitably comprises a pump operated by the motor. All of the hydraulic components involved are preferably deviod of any non-return valve. This is of great advantage, because with valves involved there is always a risk of malfunction due to inproperly working valves, especially when long time periods pass between valve operations. The reservoir may form a fluid chamber with a variable volume, and the pump may distribute fluid from the chamber to the hydraulic means of the restriction device by reduction of the volume of the chamber and withdraw fluid from the hydraulic means to the chamber by expansion of the volume of the chamber.

The implantable internal source of energy may comprise an accumulator, preferably an electric source of energy, such as a battery having a lifetime of at least 10 years.

The apparatus may comprise a switch implanted in the patient for directly or indirectly switching the supply of energy from the internal source of energy. This solution is advantageous for embodiments of the apparatus that have a relatively high energy consumption which cannot be met by direct supply of wireless energy.

In accordance with a first alternative the switch switches between an off mode, in which the internal source of energy is not in use, and an on mode, in which the internal source of energy supplies energy for the operation of the restriction device. In this alternative, the switch is conveniently operated by the wireless energy released from the external source of energy to switch between the on and off modes. The control device, preferably comprising a wireless remote control, may control the external source of energy to release the wireless energy. The advantage of this alternative is that the lifetime of the implanted source of energy, such as a battery, can be significantly prolonged, since the implanted internal source of energy does not supply energy when the switch is in its off mode.

In accordance with a second alternative , the control device comprises a wireless remote control for controlling the internal source of energy. In this case, the switch is operable by the wireless energy from the external source of energy to switch between an off mode, in which the internal source of energy and remote control are not in use, and a standby mode, in which the remote control is permitted to control the internal source of energy to supply energy for the operation of the restriction device.

In a third alternative , the apparatus further comprises an energy transforming device implantable in the patient for transforming the wireless energy into storable energy to be stored in the internal source of energy. The apparatus may further comprise an additional internal source of energy implantable in the patient for supplying energy for the operation of the restriction device, wherein the switch is operable by energy from the implantable internal source of energy to switch between an "off" mode, in which the additional internal source of energy is not in use, and an "on" mode, in which the additional internal source of energy supplies energy for the operation of the restriction device. In this alternative, the control device controls the internal source of energy to to operate the switch.

The internal source of energy preferably comprises an electric accumulator, at least one capacitor or at least one rechargeable battery, or a combination of at least one capacitor and at least one rechargeable battery.

The internal source of energy preferably comprises an electric source of energy, such as an accumulator or a battery having a lifetime of at least 10 years. However, other kinds of sources are also conceivable, such as a nuclear source of energy or a chemical source of energy.

All of the above embodiments may be combined with at least one implantable sensor for sensing at least one physical parameter of the patient, wherein the control device may control the restriction device in response to signals from the sensor. For example, the sensor may comprise a pressure sensor for directly or indirectly sensing the pressure in the colon or rectum. The expression "*indirectly sensing the pressure in the colon* or *rectum"* should be understood to encompass the cases where the sensor senses the pressure against the restriction device or human tissue of the patient. Where the control device comprises an internal control unit to be implanted in the patient, the internal control unit may suitably directly control the restriction device in response to signals from the sensor. In response to signals from the sensor, for example pressure, the patient's position or any other important physical parameter, the internal control unit may send information thereon to outside the patient's body. The control unit may also automatically control the restriction device in response to signals from the sensor. For example, the control unit may control the restriction device to firmly close the faecal passageway in response to the sensor sensing that the patient is lying down, or enlarge the faecal passageway in response to the sensor sensing an abnormally high pressure against the restriction device.

Where the control device comprises an external control unit outside the patient's body, the external control unit may, suitably directly, control the restriction device in response to signals from the sensor. The external control unit may store information on the physical parameter sensed by the sensor and may be manually operated to control the restriction device based on the stored information. In addition, there may be at least one implantable sender for sending information on the physical parameter sensed by the sensor.

An external data communicator may be provided outside the patient's body and an internal data communicator to be implanted in the patient may be provided for communicating with the external data communicator. The internal data communicator may feed data related to the patient, or related to the restriction device, back to the external data communicator. Alternatively or in combination, the external data communicator may feed data to the internal data communicator. The internal data communicator may suitably feed data related to at least one physical signal of the patient.

The restriction device may be operable to open and close the faecal passageway or may steplessly control the restriction of the faecal passageway. A pressure sensor may be provided for directly or indirectly sensing the pressure in the colon or rectum. The control device may control the restriction device in response to signals from the pressure sensor.

The apparatus may comprise an implantable energy transforming device, wherein the control device releases electric energy and the energy transforming device transforms the electric energy into kinetic energy for, preferably direct, operation of the restriction device. Suitably, an implantable stabiliser, such as a capacitor or a rechargeable accumulator, or the like, may be provided for stabilising the electric energy released by the control device. In addition, the control device may control the external source of energy to release energy for a determined time period or in a determined number of energy pulses. Finally, the restriction device may be non-inflatable.

All of the above embodiments are preferably remote controlled. Thus, the control device advantageously comprises a wireless remote control transmitting at least one wireless control signal for controlling the restriction device. With such a remote control it will be possible to adapt the function of the apparatus to the patient's need in a daily basis, which is beneficial with respect to the treatment of the patient.

The wireless remote control may be capable of obtaining information on the condition of the restriction device and of controlling the restriction device in response to the information. Also, The remote control may be capable of sending information related to the restriction device from inside the patient's body to the outside thereof.

In a particular embodiment of the invention, the wireless remote control comprises at least one external signal transmitter or transceiver and at least one internal signal receiver or transceiver implantable in the patient. In another particular embodiment of the invention, the wireless remote control comprises at least one external signal reciever or transceiver and at least one internal signal transmitter or transceiver implantable in the patient.

The remote control may transmit a carrier signal for carrying the control signal, wherein the carrier signal is frequency, amplitude or frequency and amplitude modulated and is digital, analog or digital and analog. Also the control signal used with the carrier signal may be frequency, amplitude or frequency and amplitude modulated.

The control signal may comprise a wave signal, for example, a sound wave signal, such as an ultrasound wave signal, an electromagnetic wave signal, such as an infrared light signal, a visible light signal, an ultra violet light signal, a laser signal, a micro wave signal, a radio wave signal, an x-ray radiation signal, or a gamma radiation signal. Where applicable, two or more of the above signals may be combined.

The control signal may be digital or analog, and may comprise an electric or magnetic field. Suitably, the wireless remote control may transmit an electromagnetic carrier wave signal for carrying the digital or analog control signal. For example, use of an analog carrier wave signal carrying a digital control signal would give safe communication. The control signal may be transmitted in pulses by the wireless remote control.

In all of the above solutions, the control device advantageously releases energy from the source of energy in a non-invasive, magnetic, non-magnetic, mechanical or non-mechanical manner.

The control device may release magnetic, electromagnetic, kinetic, sonic or thermal energy, or non-magnetic, non-sonic, non-thermal, non-electromagnetic or non-kinetic energy.

The control device may be activated in a manual or non-manual manner to control the source of energy to release energy.

The operation device may be powered by magnetic energy, non-magnetic energy, electromagnetic energy, non-electromagnetic energy, kinetic energy, non-kinetic energy, thermal energy or non-thermal energy. However, preferably the operation device comprises an electrical operation device.

Typically the apparatus of the invention comprises an adjustment device for adjusting the restriction of the faecal passageway. The adjustment device may be adapted to mechanically adjust the restriction device. Alternatively, the adjustment device may be adapted to hydraulically adjust the restriction device by using hydraulic means which is devoid of hydraulic fluid of the kind having a viscosity that substantially increases when exposed to heat or a magnetic field, i.e. the hydraulic fluid would not become more viscous when exposed to heat or influenced by magnetic forces.

The above-presented embodiments of the invention may be modified in accordance with the following suggestions. The released energy may comprise electric energy and an implantable capacitor having a capacity less than 0,1 µF may be provided for producing the above-mentioned train of energy pulses.

An implantable motor or pump may be provided for operating the restriction device, wherein the control device is adapted to control the external or internal source of energy to directly power the motor or pump with the released energy. Specifically, the control device may be adapted to release wireless energy in the form of a magnetic field or electromagnetic waves (excluding radio waves) for direct power of the motor or pump, as the wireless energy is being released. Where a pump is used it preferably is not a plunger type of pump.

Generally, the wireless energy comprises a signal.

The apparatus may further comprise an implantable energy transforming device for transforming wireless energy directly or indirectly into energy different than the wireless energy, for operation of the restriction device. For example, the motor or pump may be powered by the transformed energy.

The energy transforming device may transform the wireless energy in the form of sound waves, preferably directly, into electric energy for operation of the restriction device. The energy transforming device may comprise a capacitor adapted to produce electric pulses from the transformed electric energy.

The motor mentioned in the present specification may also be directly powered with wirelessly transmitted electromagnetic or magnetic energy in the form of signals, as the energy is transmitted. Furthermore, all the various functions of the motor and associated components described in the present specification may be used where applicable.

Generally, the restriction device advantageously is embedded in a soft or gel-like material, such as a silicone material having hardness less than 20 Shore.

Of course, the restriction device preferably is adjustable in a non-manual manner.

All the above described various components, such as the motor, pump and capacitor, may be combined in the different embodiments where applicable. Also the various functions described in connection with the above embodiments of the invention may be used in different applications, where applicable.

All the various ways of transferring energy and controlling the energy presented in the present specification may be practised by using all of the various components and solutions described.

There are also provided methods for treating anal incontinent patients. These methods do not form part of the invention but are useful for understanding the invention.

Accordingly, in accordance with a first alternative method, there is provided a method of treating a patient suffering from anal incontinence, comprising the steps of implanting an operable restriction device in the patient, so that the restriction device engages the colon or rectum to form a restricted faecal passageway in the colon or rectum, providing a source of energy for energizing the restriction device, and controlling the source of energy to release energy for use in connection with the operation of the restriction device. The method may further comprise using energy released from the source of energy to operate the restriction device to open and close, respectively, the faecal passageway.

In accordance with a second alternative method, there is provided a method of treating a patient suffering from anal incontinence, comprising the steps of placing at least two laparascopical trocars in the patient's body, inserting a dissecting tool through the trocars and dissecting an area of the colon or rectum, placing an operable restriction device in the dissected area, so that the restriction device engages the colon or rectum to form a restricted faecal passageway in the colon or rectum, implanting a source of energy in the patient, and controlling the implanted source of energy from outside the patient's body to release energy for use in connection with the operation of the restriction device.

In accordance with a third alternative method, there is provided a method of treating a patient suffering from anal incontinence, comprising: (a) Surgically implanting in the patient an operable restriction device engaging the patient's colon or rectum to form a restricted faecal passageway in the colon or rectum. (b) Providing a source of energy external to the patient's body. (c) Controlling the external source of energy from outside the patient's body to release wireless energy. And (d) using the released wireless energy in connection with the operation of the restriction device.

The method may further comprise (e) implanting in the human or animal an operation device which can adjust the restricted faecal passageway in response to supplied energy, and (f) using the released wireless energy to activate the implanted operation device so as (i) to enlarge the restricted faecal passageway to allow feaces to readily pass therethrough but normally restrict the faecal passageway.

In accordance with a fourth alternative method, there is provided a method of treating a patient suffering from anal incontinence, comprising the steps of placing at least two laparascopical trocars in the patient's body, inserting a dissecting tool through the trocars and dissecting an area of the colon or rectum, placing an operable restriction device in the dissected area, so that the restriction device engages the colon or rectum to form a restricted faecal passageway in the colon or rectum, providing an external source of energy outside the patient's body, controlling the external source of energy from outside the patient's body to release wireless energy, and using the released wireless energy in connection with the operation of the restriction device.

In accordance with a fifth alternative method, there is provided a method of treating a patient suffering from anal incontinence, comprising the steps of placing at least two laparascopical trocars in the patient's body, inserting a dissecting tool through the trocars and dissecting an area of the colon or rectum, implanting an operable restriction device in the dissected area, so that the restriction device engages the colon or rectum to form a restricted faecal passageway in the colon or rectum, implanting an energy transforming device, providing an external source of energy, controlling the external source of energy to release wireless energy, and transforming the wireless energy by the energy transforming device into energy different than the wireless energy for use in connection with the operation of the restriction device. This method may further comprise implanting a stabiliser in the patient for stabilising the energy transformed by the energy transforming device.

The invention is described in more detail in the following with reference to the accompanying drawings, in which
FIGURES 1 to 6 are schematic block diagrams illustrating six embodiments, respectively, of the invention, in which wireless energy released from an external source of energy is used for direct operation of a restriction device engaging the colon or rectum of a patient;
FIGURES 7 to 10 are schematic block diagrams illustrating four embodiments, respectively, of the invention, in which energy is released from an implanted source of energy;
FIGURES 11 to 15 are schematic block diagrams illustrating five embodiments, respectively, of the invention, in which a switch is implanted in the patient for directly or indirectly switching the operation of the restriction device;
FIGURE 16 is a schematic block diagram illustrating conceivable combinations of implantable components for achieving various communication options;
FIGURE 17 illustrates the apparatus in accordance with the invention implanted in a patient;
FIGURE 18 is a block diagram illustrating remote control components of an embodiment of the invention; and
FIGURE 19 is a schematic view of exemplary circuitry used for the components of the block diagram of FIGURE 18.

Referring to the drawing figures, like reference numerals designate identical or corresponding elements throughout the several figures.

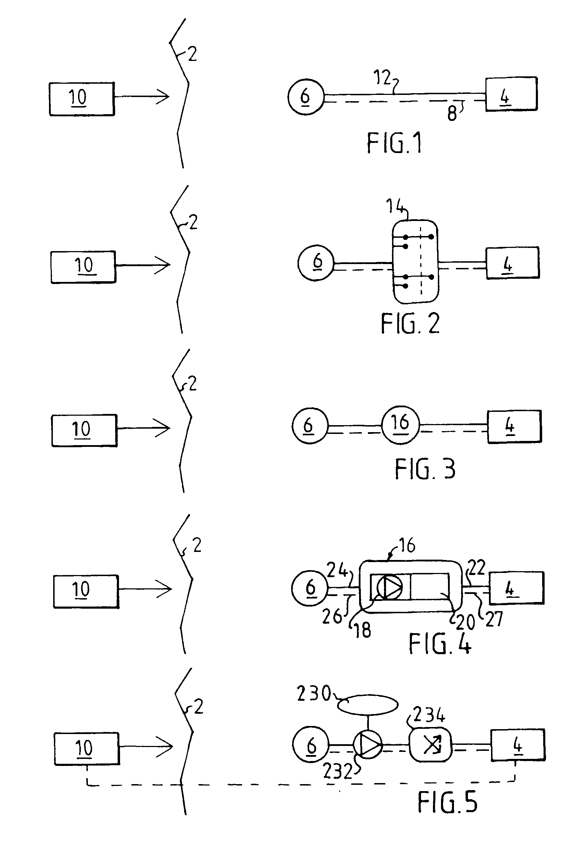

FIGURE 1 schematically shows an embodiment of the anal incontinence treatment apparatus of the invention having some parts implanted in a patient and other parts located outside the patient's body. Thus, in FIGURE 1 all parts placed to the right of the patient's skin 2 are implanted and all parts placed to the left of the skin 2 are located outside the patient's body. The apparatus of FIGURE 1 comprises an implanted operable restriction device 4, which engages the patient's colon (or alternatively the rectum or anus) to form a restricted faecal passageway in the colon. The restriction device 4 is capable of performing a reversible function, i.e. to open and close the faecal passageway. An implanted control unit 6 controls the restriction device 4 via a control line 8 to form an adequate restriction of the faecal passageway. An external control unit 10 includes an external source of energy and a wireless remote control transmitting a control signal generated by the external source of energy. The control signal is received by a signal receiver incorporated in the implanted control unit 6, whereby the control unit 6 controls the implanted restriction device 4 in response to the control signal. The implanted control unit 6 also uses energy from the control signal for operating the restriction device 4 via a power supply line 12.

FIGURE 2 shows an embodiment of the invention identical to that of FIGURE 1, except that a reversing device in the form of a switch 14 operable by energy also is implanted in the patient for reversing the restriction device 4. The control unit 6 uses the switch 14 to reverse the function performed by the restriction device 4. More precisely, the external control unit 10 releases energy carried by a wireless signal and the implanted control unit 6 transforms the wireless energy into a current for operating the switch 14. When the control unit 6 shifts the polarity of the current the switch 14 reverses the function performed by the restriction device 4.

FIGURE 3 shows an embodiment of the invention identical to that of FIGURE 1, except that an operation device in the form of a motor 16 also is implanted in the patient. The implanted control unit 6 powers the motor 16 with wireless energy released from the external source of energy of the external control unit 10. The implanted control unit 6 controls the operation of the motor 16 in response to a control signal from the remote control of the external control unit 10.

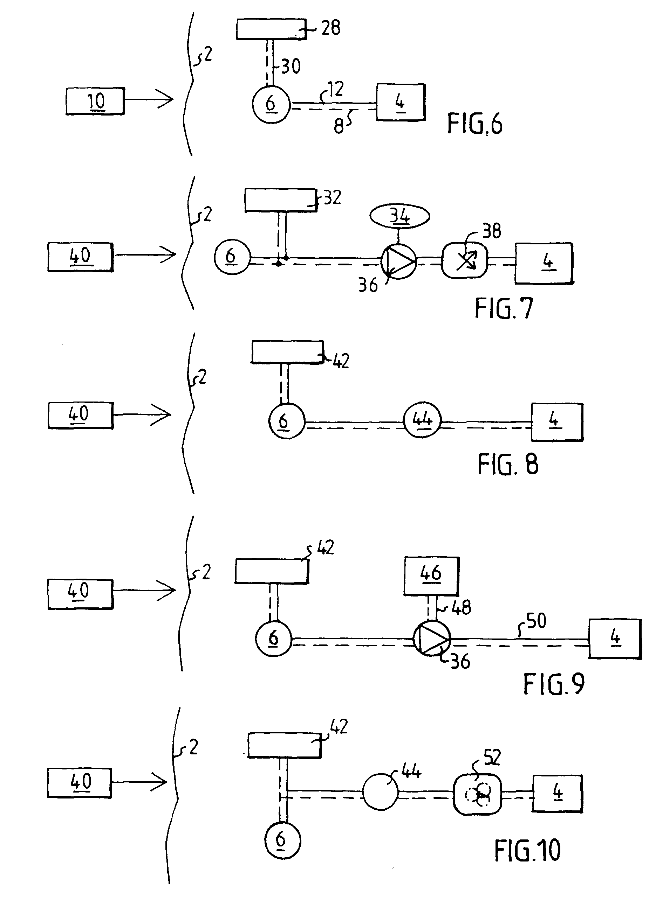

FIGURE 4 shows an embodiment of the invention identical to that of FIGURE 1, except that an assembly 16 including a motor/pump unit 18 and a fluid reservoir 20 also is implanted in the patient. In this case the restriction device 4 is hydraulically operated, i.e. hydraulic fluid is pumped by the motor/pump unit 18 from the reservoir 20 through a conduit 22 to the restriction device 4 to restrict the faecal passageway, and hydraulic fluid is pumped by the motor/pump unit 18 back from the restriction device 4 to the reservoir 20 to enlarge the faecal passageway. The external control unit 10 releases energy carried by a wireless signal and the implanted control unit 6 transforms the wireless energy into a current, for example a current, for powering the motor/pump unit 18 via an electric power supply line 24. The implanted control unit 6 controls the motor/pump unit 16 and the restriction device 4 via control lines 26 and 27.

FIGURE 5 shows an embodiment of the invention comprising the restriction device 4, hydraulically operated, and the implanted control unit 6, and further comprising a hydraulic fluid reservoir 230, a motor/pump unit 232 and a reversing device in the form of a hydraulic valve shifting device 234, all of which are implanted in the patient. The motor of the motor/pump unit 232 is an electric motor.

FIGURE 6 shows an embodiment of the invention identical to that of FIGURE 1, except that an internal source of energy in the form of an accumulator 28 also is implanted in the patient. The control unit 6 stores energy received from the external control unit 10 in the accumulator 28. In response to a control signal from the external control unit 10 the implanted control unit 6 releases energy from the accumulator 28 via a power line 30 for the operation of the restriction device 4.

FIGURE 7 shows an embodiment of the invention comprising the restriction device 4, hydraulically operated, and the implanted control unit 6, and further comprising a source of energy in the form of a battery 32, a hydraulic fluid reservoir 34, a motor/pump unit 36 and a reversing device in the form of a hydraulic valve shifting device 38, all of which are implanted in the patient. The motor of the motor/pump unit 36 is an electric motor. An external control unit 40 includes a wireless remote control transmitting a control signal which is received by the signal receiver incorporated in the implanted control unit 6.

In response to a control signal from the external control unit 40 the implanted control unit 6 powers the motor/pump unit 36 with energy from the battery 32, whereby the motor/pump unit 36 distributes hydraulic fluid between the reservoir 34 and the restriction device 4. The control unit 6 controls the shifting device 38 to shift the hydraulic fluid flow direction between one direction in which the fluid is pumped by the motor/pump unit 36 from the reservoir 34 to the restriction device 4 to restrict the faecal passageway, and another opposite direction in which the fluid is pumped by the motor/pump unit 36 back from the restriction device 4 to the reservoir 34 to enlarge the faecal passageway.

FIGURE 8 shows an embodiment of the invention identical to that of FIGURE 6, except that a battery 42 is substituted for the accumulator 28, the external control unit 40 of the embodiment of FIGURE 5 is substituted for the external control unit 10 and an electric motor 44 is implanted in the patient for operating the restriction device 4. In response to a control signal from the external control unit 40 the implanted control unit 6 powers the motor 44 with energy from the battery 42, whereby the motor 44 operates the restriction device 4.

FIGURE 9 shows an embodiment of the invention identical to that of FIGURE 8, except that the motor/pump unit 36 of the embodiment of FIGURE 7 is substituted for the motor 44 and a fluid reservoir 46 also is implanted in the patient. The reservoir 46 is via fluid conduits 48 and 50 connected to the motor/pump unit 36 and restriction device 4, which in this case is hydraulically operated. In response to a control signal from the external control unit 40, the implanted control unit 6 powers the electric motor of the motor/pump unit 36 with energy from the battery 42, whereby the motor/pump unit 36 distributes hydraulic fluid between the fluid reservoir 46 and the restriction device 4.

FIGURE 10 shows an embodiment of the invention identical to that of FIGURE 8, except that a mechanical reversing device in the form of a gearbox 52 also is implanted in the patient. The implanted control unit 6 controls the gearbox 52 to reverse the function performed by the restriction device 4 (mechanically operated).

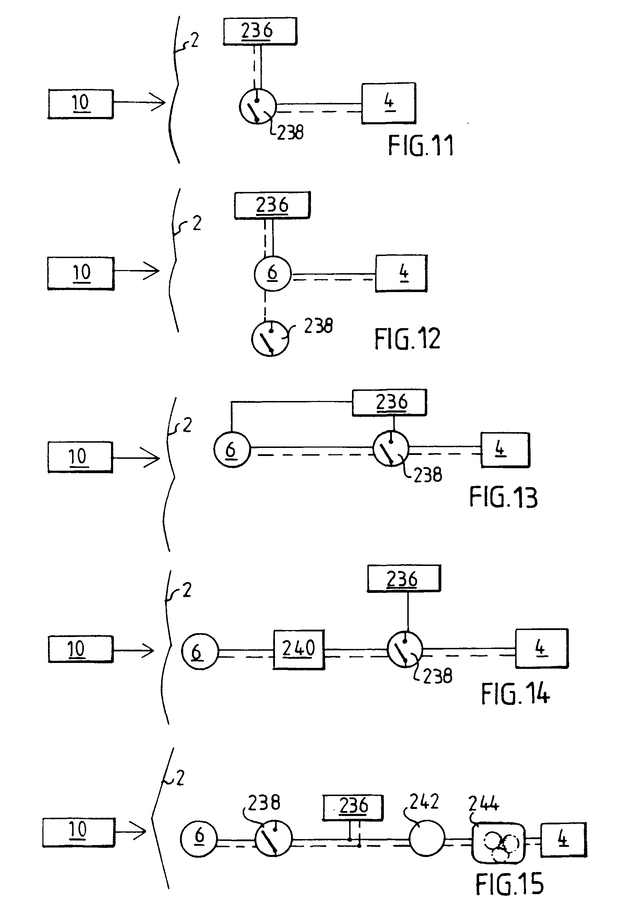

FIGURE 11 shows an embodiment of the invention comprising the restriction device 4, the external control unit 10, an implanted internal source of energy 236 and an implanted switch 238. The switch 238 is operated by wireless energy released from the external source of energy of the external control unit 6 to switch between an off mode, in which the implanted source of energy 236 is not in use, and an on mode, in which the implanted source of energy 236 supplies energy for the operation of the restriction device 4.

FIGURE 12 shows an embodiment of the invention identical to that of FIGURE 11, except that also the control unit 6 is implanted, in order to receive a control signal from the wireless remote control of the external control unit 10. The switch 238 is operated by the wireless energy from the external source of energy 10 to switch between an off mode, in which the implanted source of energy 236 and the wireless remote control of the external control unit 10 are not in use, i.e. the control unit 6 is not capable of receiving the control signal, and a standby mode, in which the wireless remote control is permitted to control the internal source of energy 236, via the implanted control unit 6, to supply energy for the operation of the restriction device 4.

FIGURE 13 shows an embodiment of the invention identical to that of FIGURE 12, except that an energy transforming device for transforming the wireless energy into storable energy is incorporated in the implanted control unit 6 and that the implanted internal source of energy 236 is of a type that is capable of storing the storable energy. In this case, in response to a control signal from the external control unit 10, the implanted control unit 6 controls the switch 238 to switch from an off mode, in which the implanted source of energy 236 is not in use, to an on mode, in which the source of energy 36 supplies energy for the operation of the restriction device 4.

FIGURE 14 shows an embodiment of the invention identical to that of FIGURE 13, except that an energy storage device 240 also is implanted in the patient for storing the storable energy transformed from the wireless energy by the transforming device of the control unit 6. In this case, the implanted ontrol unit 6 controls the energy storage device 240 to operate the switch 238 to switch between an off mode, in which the implanted source of energy 236 is not in use, and an on mode, in which the implanted source of energy 236 supplies energy for the operation of the restriction device 4.

FIGURE 15 shows an embodiment of the invention identical to that of FIGURE 13, except that a motor 242 and a mechanical reversing device in the form of a gearbox 244 also are implanted in the patient. The implanted control unit 6 controls the gearbox 244 to reverse the function performed by the restriction device 4 (mechanically operated), i.e. enlarging and restricting the faecal passageway.

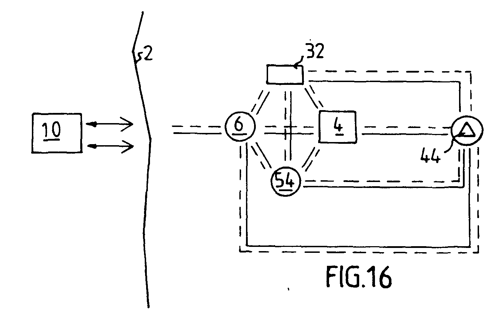

FIGURE 16 schematically shows conceivable combinations of implanted components of the apparatus for achieving various communication possibilities. Basically, there are the implanted restriction device 4, the implanted control unit 6 and the external control unit 10 including the external source of energy and the wireless remote control. As already described above the remote control transmits a control signal generated by the external source of energy, and the control signal is received by a signal receiver incorporated in the implanted control unit 6, whereby the control unit 6 controls the implanted restriction device 4 in response to the control signal.

A sensor 54 may be implanted in the patient for sensing a physical parameter of the patient, such as the pressure in the stomach. The control unit 6, or alternatively the external control unit 10, may control the restriction device 4 in response to signals from the sensor 54. A transceiver may be combined with the sensor 54 for sending information on the sensed physical parameter to the external control unit 10. The wireless remote control of the external control unit 10 may comprise a signal transmitter or transceiver and the implanted control unit 6 may comprise a signal receiver or transceiver. Alternatively, the wireless remote control of the external control unit 10 may comprise a signal receiver or transceiver and the implanted control unit 6 may comprise a signal transmitter or transceiver. The above transceivers, transmitters and receivers may be used for sending information or data related to the restriction device from inside the patient's body to the outside thereof.

The motor 44 may be implanted for operating the restriction device 4 and also the battery 32 may be implanted for powering the motor 44. The battery 32 may be equipped with a transceiver for sending information on the charge condition of the battery.

Those skilled in the art will realize that the above various embodiments according to FIGURES 1-15 could be combined in many different ways. For example, the energy operated switch 14 could be incorporated in any of the embodiments of FIGURES 4,6,8-10. The hydraulic shifting device 38 could be incorporated in any of the embodiments of FIGURES 4 and 9. The gearbox 52 could be incorporated in any of the embodiments of FIGURES 1,6 and 8.

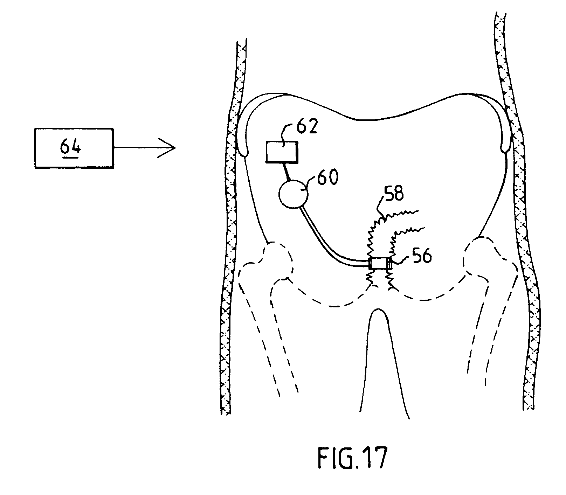

FIGURE 17 illustrates how any of the above-described embodiments of the apparatus of the invention may be implanted in a patient. Thus, an assembly of the apparatus implanted in the patient comprises a restriction device 56 engaging the rectum 58, an operation device 60 for operating the restriction device 56 and an internal control unit 62, which includes a signal receiver, for controlling the operation device 60. An external control unit 64 includes a signal transmitter for transmitting a wireless control signal to the signal receiver of the implanted control unit 62. The implanted control unit 62 is capable of transforming signal energy from the control signal into electric energy for powering the operation device 60 and for energizing energy consuming implanted components of the apparatus.

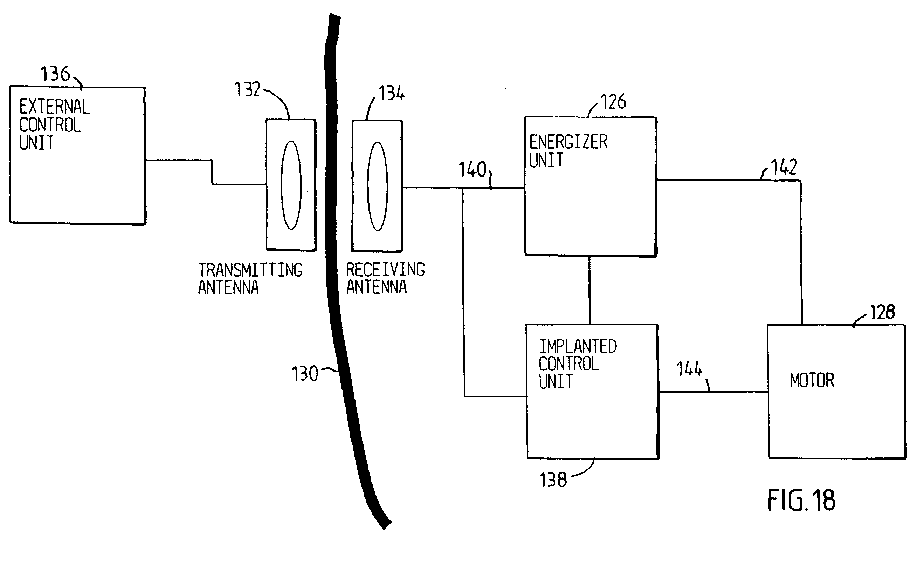

FIGURE 18 shows the basic parts of a wireless remote control of the apparatus of the invention including an electric motor 128 for operating a restriction member, for example of the type illustrated in FIGURE 17. In this case, the remote control is based on the transmission of electromagnetic wave signals, often of high frequencies in the order of 100 kHz - 1 gHz, through the skin 130 of the patient. In FIGURE 18, all parts placed to the left of the skin 130 are located outside the patient's body and all parts placed to the right of the skin 130 are implanted. Any suitable remote control system may be used.

An external signal transmitting antenna 132 is to be positioned close to a signal receiving antenna 134 implanted close to the skin 130. As an alternative, the receiving antenna 134 may be placed for example inside the abdomen of the patient. The receiving antenna 134 comprises a coil, approximately 1-100 mm, preferably 25 mm in diameter, wound with a very thin wire and tuned with a capacitor to a specific high frequency. A small coil is chosen if it is to be implanted under the skin of the patient and a large coil is chosen if it is to be implanted in the abdomen of the patient. The transmitting antenna 132 comprises a coil having about the same restriction as the coil of the receiving antenna 134 but wound with a thick wire that can handle the larger currents that is necessary. The coil of the transmitting antenna 132 is tuned to the same specific high frequency as the coil of the receiving antenna 134.

An external control unit 136 comprises a microprocessor, a high frequency electromagnetic wave signal generator and a power amplifier. The microprocessor of the control unit 136 is adapted to switch the generator on/off and to modulate signals generated by the generator to send digital information via the power amplifier and the antennas 132,134 to an implanted control unit 138. To avoid that accidental random high frequency fields trigger control commands, digital signal codes are used. A conventional keypad placed on the external control unit 136 is connected to the microprocessor thereof. The keypad is used to order the microprocessor to send digital signals to either contract or enlarge the restriction device. The microprocessor starts a command by applying a high frequency signal on the antenna 132. After a short time, when the signal has energized the implanted parts of the control system, commands are sent to contract or enlarge the restriction device in predefined steps. The commands are sent as digital packets in the form illustrated below.

| | | | |
|---|---|---|---|
| Start pattern, 8 bits | Command, 8 bits | Count, 8 bits | Checksum, 8 bits |

The commands are sent continuously during a rather long time period (e.g. about 30 seconds or more). When a new contract or enlarge step is desired the Count byte is increased by one to allow the implanted control unit 138 to decode and understand that another step is demanded by the external control unit 136. If any part of the digital packet is erroneous, its content is simply ignored.

Through a line 140, an implanted energizer unit 126 draws energy from the high frequency electromagnetic wave signals received by the receiving antenna 134. The energizer unit 126 stores the energy in a power supply, such as a large capacitor, powers the control unit 138 and powers the electric motor 128 via a line 142.

The control unit 138 comprises a demodulator and a microprocessor. The demodulator demodulates digital signals sent from the external control unit 136. The microprocessor of the control unit 138 receives the digital packet, decodes it and, provided that the power supply of the energizer unit 126 has sufficient energy stored, sends a signal via a signal line 144 to the motor 128 to either contract or enlarge the restriction device depending on the received command code.

Alternatively, the energy stored in the power supply of the energizer unit may only be used for powering a switch, and the energy for powering the motor 128 may be obtained from another implanted power source of relatively high capacity, for example a battery. In this case the switch is adapted to connect said battery to the control unit 138 in an on mode when said switch is powered by said power supply and to keep said battery disconnected from the control unit in a standby mode when said switch is unpowered.

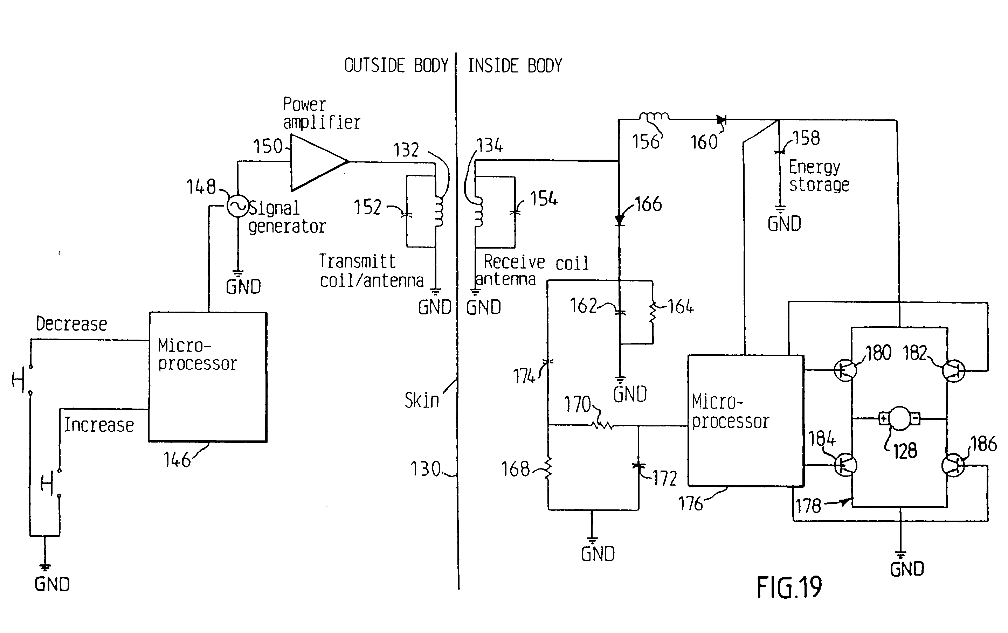

With reference to FIGURE 19, the remote control schematically described above will now be described in accordance with a more detailed embodiment. The external control unit 136 comprises a microprocessor 146, a signal generator 148 and a power amplifier 150 connected thereto. The microprocessor 146 is adapted to switch the signal generator 148 on/off and to modulate signals generated by the signal generator 148 with digital commands that are sent to implanted components of the apparatus. The power amplifier 150 amplifies the signals and sends them to the external signal transmitting antenna 132. The antenna 132 is connected in parallel with a capacitor 152 to form a resonant circuit tuned to the frequency generated by the signal generator 148.

The implanted signal receiving antenna coil 134 forms together with a capacitor 154 a resonant circuit that is tuned to the same frequency as the transmitting antenna 132. The signal receiving antenna coil 134 induces a current from the received high frequency electromagnetic waves and a rectifying diode 160 rectifies the induced current, which charges a storage capacitor 158. A coil 156 connected between the antenna coil 134 and the diode 160 prevents the capacitor 158 and the diode 160 from loading the circuit of the signal receiving antenna 134 at higher frequencies. Thus, the coil 156 makes it possible to charge the capacitor 158 and to transmit digital information using amplitude modulation.

A capacitor 162 and a resistor 164 connected in parallel and a diode 166 forms a detector used to detect amplitude modulated digital information. A filter circuit is formed by a resistor 168 connected in series with a resistor 170 connected in series with a capacitor 172 connected in series with the resistor 168 via ground, and a capacitor 174, one terminal of which is connected between the resistors 168,170 and the other terminal of which is connected between the diode 166 and the circuit formed by the capacitor 162 and resistor 164. The filter circuit is used to filter out undesired low and high frequencies. The detected and filtered signals are fed to an implanted microprocessor 176 that decodes the digital information and controls the motor 128 via an H-bridge 178 comprising transistors 180,182,184 and 186. The motor 128 can be driven in two opposite directions by the H-bridge 178.

The microprocessor 176 also monitors the amount of stored energy in the storage capacitor 158. Before sending signals to activate the motor 128, the microprocessor 176 checks whether the energy stored in the storage capacitor 158 is enough. If the stored energy is not enough to perform the requested operation, the microprocessor 176 waits for the received signals to charge the storage capacitor 158 before activating the motor 128.

## Claims

1. An anal incontinence treatment apparatus, comprising:
a restriction device (4, 56) for engaging the colon (58) or rectum to form a restricted faecal passageway in the colon (58) or rectum, the restriction device (4, 56) being operable to change the restriction of the faecal passageway,
an internal source of energy (32, 42, 236) implantable in the patient, and
a control device (6, 10) operable from outside the patient's body for controlling the internal source of energy (32, 42, 236) to release energy for use in connection with the operation of the restriction device (4, 56),
**characterized in that** the apparatus comprises:
an external source of energy (10, 40) intended to be external to the patient's body when the restriction device (4, 56) is implanted therein, the external source of energy (10, 40) being adapted to release wireless energy, and
an implantable energy transforming device (6, 62) for transforming the released wireless energy into storable energy, and wherein
the internal source of energy (32, 42, 236) is capable of storing the storable energy, and
the control device (6, 10) is adapted to control the internal source of energy (32, 42, 236) to supply energy for the operation of the restriction device (4, 56), and the control device (6, 10) comprises:
a first control unit (136) operable from outside the patient's body for providing a control signal, and
a second control unit (138) implantable in the patient adapted to control the restriction device (4, 56) in response to the control signal.

2. An apparatus according to claim 1, wherein the second control unit (138) is programmable.

3. An apparatus according to claim 2, wherein the first control unit (136) is intended to be outside the patient's body, the first control unit (136) being programmable by the second control unit (138).

4. An apparatus according to claim 2, wherein the second control unit (138) is programmable for controlling the restriction device (4, 56) over time.

5. An apparatus according to claim 4, wherein the second control unit (138) controls the restriction device (4, 56) over time in accordance with an activity schedule program.

6. An apparatus according to claim 4, wherein the second control unit (138) comprises a microprocessor (146).

7. An apparatus according to claim 3, wherein the first control unit (136) loads the second control unit (138) with data in accordance with a loading mode only authorized for a doctor.

8. An apparatus according to claim 3, wherein the first control unit (136) controls the second control unit (138) in accordance with a doctor mode only authorized for a doctor.

9. An apparatus according to claim 3, wherein the first control unit controls (136) the second control unit (138) in accordance with a patient mode permitted for the patient.

10. An apparatus according to claim 1, wherein the internal source of energy (32, 42, 236) comprises an accumulator (28).

11. An apparatus according to claim 10, wherein the accumulator (28) comprises an electric accumulator.

12. An apparatus according to claim 11, wherein the electric accumulator comprises at least one capacitor or at least one rechargeable battery, or a combination of at least one capacitor and at least one rechargeable battery.

13. An apparatus according to claim 1, further comprising a battery implantable in the patient for supplying electric energy to implantable electric energy consuming components of the apparatus.

14. The apparatus according to claim 1, wherein the control device (6, 10) is adapted to control the external source of energy (10, 40) to release wireless energy for direct use in connection with the operation of the restriction device (4, 56).

15. The apparatus according to claim 14, wherein the control device (6, 10) is adapted to control the external source of energy (10, 40) to intermittently release wireless energy in the form of a train of energy pulses for direct use in connection with the operation of the restriction device (4, 56).

16. The apparatus according to claim 14 or 15, wherein the restriction device (6, 10) is operable in a non-magnetic, non-thermal or non-mechanical manner by use of said released wireless energy.

17. The apparatus according to claim 1, wherein the external source of energy (10, 40) comprises a nuclear source of energy.

18. The apparatus according to claim 1, wherein the external source of energy (10, 40) comprises a chemical source of energy.

19. An apparatus according to claim 1, further comprising an operation device (60) implantable in the patient for operating the restriction device (4, 56).

20. An apparatus according to claim 19, wherein the wireless energy directly or indirectly powers the operation device (60).

21. An apparatus according to claim 19 or 20, wherein the control device (6, 10) controls the operation device (60) to operate the restriction device (4, 56).

22. An apparatus according to claim 21, wherein the operation device (60) comprises hydraulic means and at least one valve for controlling a fluid flow in the hydraulic means.

23. An apparatus according to claim 22, wherein the control device (6, 10) comprises a wireless remote control for controlling the valve.

24. An apparatus according to claim 21, wherein the restriction device (4, 56) comprises hydraulic means and the operation device (60) comprises a reservoir (20, 34, 46, 230) forming a fluid chamber with a variable volume connected to the hydraulic means, and the operation device (60) is adapted to distribute fluid from the chamber to the hydraulic means by reduction of the volume of the chamber and to withdraw fluid from the hydraulic means to the chamber by expansion of the volume of the chamber.

25. An apparatus according to claim 19 or 21, wherein the operation device (60) comprises a motor (16, 18, 36, 232).

26. An apparatus according to claim 25, wherein the motor (16, 18, 36, 232) comprises a rotary motor, and the control device (6, 10) controls the rotary motor to rotate a desired number of revolutions.

27. An apparatus according to claim 25, wherein the motor (16, 18, 36, 232) comprises a linear motor.

28. An apparatus according to claim 25, wherein the motor (16, 18, 36, 232) comprises a hydraulic or pneumatic fluid motor, and the control device (6, 10) controls the fluid motor.

29. An apparatus according to claim 25, wherein the motor (16, 18, 36, 232) comprises an electric motor having electrically conductive parts made of plastics.

30. An apparatus according to any one of claims 19-21, wherein the control device (6, 10) releases polarized energy from the internal source of energy (32, 42, 236).

31. An apparatus according to any one of claims 19-21, wherein the control device (6, 10) shifts polarity of the released energy to reverse the operation device (60).

32. An apparatus according to any one of claims 19-21, wherein the operation device (60) comprises an electric motor (232) and the released energy comprises electric energy.

33. An apparatus according to any one of claims 19-21 and 25, wherein the restriction device (4, 56) is operable to perform a reversible function.

34. An apparatus according to claim 33, further comprising a reversing device (14, 38, 52, 234) implantable in the patient for reversing the function performed by the restriction device (4, 56).

35. An apparatus according to claim 34, wherein the control device (6, 10) controls the reversing device (14, 38, 52, 234) to reverse the function performed by the restriction device (4, 56).

36. An apparatus according to claim 34, wherein the reversing device (14, 38, 52, 234) comprises hydraulic means including a valve (38) for shifting the flow direction of a fluid in the hydraulic means.

37. An apparatus according to claim 36, wherein the reversing device (14, 38, 52, 234) comprises a mechanical reversing device (14, 52).

38. An apparatus according to claim 37, wherein the mechanical reversing device comprises a switch (14).

39. An apparatus according to claim 37, wherein the reversing device (14, 38, 52, 234) comprises a gearbox (52).

40. An apparatus according to claim 34, wherein the reversing device (14, 38, 52, 234) comprises a switch (14).

41. An apparatus according to claim 40, wherein the switch (14) of the reversing device is operable by the released energy.

42. An apparatus according to claim 41, wherein the control device (6, 10) controls the operation of the switch (14) of the reversing device by shifting polarity of the released energy supplied to the switch (14).

43. An apparatus according to claim 40, wherein the switch (14) comprises an electric switch and the internal source of energy (32, 42, 236) supplies electric energy for the operation of the switch (14).

44. An apparatus according to claim 40, wherein the operation device (60) comprises a motor (16, 18, 36, 232), and the reversing device (14, 38, 52, 234) reverses the motor (16, 18, 36, 232).

45. An apparatus according to any of claims 1, 19-21, wherein the restriction device (4, 56) comprises hydraulic means and the operation device (60) is adapted to conduct a hydraulic fluid in the hydraulic means.

46. An apparatus according to claim 45, wherein the operation device (60) comprises a motor (16, 18, 36, 232).

47. An apparatus according to claim 45 or 46, wherein the operation device (60) comprises a fluid conduit connected to the hydraulic means of the restriction device (4, 56), and a reservoir (20, 34, 46, 230) for fluid, the reservoir (20, 34, 46, 230) forming part of the conduit.

48. An apparatus according to claim 47, wherein the hydraulic means and conduit are devoid of any non-return valve.

49. An apparatus according to claim 48, wherein the reservoir (20, 34, 46, 230) forms a fluid chamber with a variable volume, and the operation device (60) is adapted to distribute fluid from the chamber to the hydraulic means of the restriction device (4, 56) by reduction of the volume of the chamber and to withdraw fluid from the hydraulic means to the chamber by expansion of the volume of the chamber.

50. An apparatus according to any of the preceding claims, further comprising at least one implantable sensor (54) for sensing at least one physical parameter of the patient.

51. An apparatus according to claim 50, wherein the sensor (54) comprises a pressure sensor for directly or indirectly sensing as the physical parameter the pressure in the colon (58) or rectum.

52. An apparatus according to claim 50 or 51, wherein the control device (6, 10) controls the restriction device (4, 56) in response to signals from the sensor (54).

53. An apparatus according to claim 52, wherein the second control unit (138) controls the restriction device (4, 56) in response to signals from the sensor (54).

54. An apparatus according to claim 53, wherein the first control unit (136) controls the restriction device (4, 56) in response to signals from the sensor (54).

55. An apparatus according to claim 54, wherein the first control unit (136) stores information on the physical parameter sensed by the sensor (54) and is manually operated to control the restriction device (4, 56) based on the stored information.

56. An apparatus according to any of claims 50-55, further comprising at least one implantable sender for sending information on the physical parameter sensed by the sensor (54).

57. An apparatus according to any of the preceding claims, further comprising an external data communicator intended to be outside the patient's body and an internal data communicator implantable in the patient for communicating with the external communicator, wherein the internal data communicator feeds data related to the patient back to the external data communicator or the external data communicator feeds data to the internal data communicator.

58. An apparatus according to claim 57, wherein the internal data communicator feeds data related to the restriction device (4, 56).

59. An apparatus according to claim 57 or 58, wherein the implantable communicator feeds data related to at least one physical signal of the patient.

60. An apparatus according to any of the preceding claims, wherein the restriction device (4, 56) is adapted to control the restriction of the faecal passageway.

61. An apparatus according to claim 60, wherein the restriction device (4, 56) is operable to enlarge and restrict the faecal passageway when implanted in the patient.

62. An apparatus according to claim 60 or 61, wherein the restriction (4, 56) device is adapted to steplessly control the restriction of the faecal passageway when implanted in the patient.

63. An apparatus according to any of the preceding claims, wherein the control device (6, 10) is adapted to control the external source of energy (10, 40) to release wireless electric energy and the energy transforming device (6, 62) is adapted to transform the electric energy into kinetic energy for operation of the restriction device (4, 56).

64. An apparatus according to claim 63, wherein the restriction device (4, 56) is directly operated with the kinetic energy, as the energy transforming device (6, 62) transforms the electric energy into the kinetic energy.

65. An apparatus according to any of the preceding claims, wherein the control device (6, 10) controls the internal source of energy (32, 42, 236) to release magnetic energy, non-magnetic energy, electromagnetic energy, non-electromagnetic energy, kinetic energy, non-kinetic energy, sonic energy, non-sonic energy, thermal energy or non-thermal energy.

66. An apparatus according to any of the preceding claims, wherein the restriction device (4, 56) is non-inflatable.

67. An apparatus according to any of the preceding claims, wherein the control device (6, 10) controls the internal source of energy (32, 42, 236) to release energy for a determined time period.

68. An apparatus according to any of claims 1-66, wherein the control device (6, 10) controls the external source of energy (10, 40) to release energy in a determined number of energy pulses.

69. An apparatus according to any of the preceding claims, wherein the control device (6, 10) is adapted to control the internal source of energy (32, 42, 236) to release energy in a non-invasive manner.

70. An apparatus according to any of the preceding claims, wherein the control device (6, 10) comprises a wireless remote control for transmitting at least one wireless control signal for controlling the restriction device (4, 56).

71. An apparatus according to claim 70, wherein the remote control is capable of obtaining information on the condition of the restriction device (4, 56) when the restriction device (4, 56) is implantable and to control the restriction device (4, 56) in response to the information.

72. An apparatus according to claim 70 or 71, wherein the wireless remote control comprises at least one external signal transmitter or transceiver and at least one internal signal receiver or transceiver implantable in the patient.

73. An apparatus according to claim 70 or 71, wherein the wireless remote control comprises at least one external signal receiver or transceiver and at least one internal signal transmitter or transceiver implantable in the patient.

74. An apparatus according to any of claims 70-73, wherein the remote control is capable of sending information related to the restriction device (4, 56) from inside the patient's body to the outside thereof.

75. An apparatus according to claim 74, wherein the remote control controls the restriction device (4, 56) in response to the information.

76. An apparatus according to any of claims 70-75, wherein the remote control transmits a carrier signal for carrying the control signal.

77. An apparatus according to claim 76, wherein the carrier signal is frequency, amplitude or frequency and amplitude modulated.

78. An apparatus according to claim 76 or 77, wherein the carrier signal is digital, analog or digital and analog.

79. An apparatus according to any of claims 76-78, wherein the control signal used with the carrier signal is frequency, amplitude or frequency and amplitude modulated.

80. An apparatus according to any of claims 70-79, wherein the control signal comprises a wave signal comprising one of a sound wave signal including an ultrasound wave signal, an electromagnetic wave signal including an infrared light signal, a visible light signal, an ultra violet light signal and a laser light signal, a micro wave signal, a radio wave signal, an x-ray radiation signal, and a gamma radiation signal.

81. An apparatus according to any of claims 70-79, wherein the control signal comprises an electric, magnetic or electric and magnetic field.

82. An apparatus according to any of claims 70-80, wherein the control signal is digital, analog or digital and analog.

83. An apparatus according to claim 82, wherein the remote control transmits an electromagnetic carrier wave signal for carrying the digital or analog control signal.

84. An apparatus according to any of claims 70-83, wherein the control signal is transmitted in pulses by the wireless remote control.

85. An apparatus according to any of claims 14-16 or 61-63, further comprising an implantable stabilizer for stabilizing the energy released by the control device (6, 10).

86. An apparatus according to claim 85, wherein the energy released by the control device (6, 10) comprises electric energy and the stabilizer comprises at least one capacitor (162).

87. An apparatus according to claim 1, wherein the restriction device (4, 56) is operable by the released energy in a manual, mechanical, thermal or magnetic manner.

88. An apparatus according to claim 1, wherein the restriction device (4, 56) is operable by the released energy in a non-manual, non-mechanical, non-thermal or non-magnetic manner.

89. An apparatus according to claim 14 or 16, wherein the control device (6, 10) is adapted to control the internal source of energy (32, 42, 236) to release electric energy, and further comprising an implantable capacitor for producing the train of energy pulses from the released energy.

90. An apparatus according to claim 1, further comprising an adjustment device for adjusting the restriction device (4, 56) to change the restriction faecal passageway, wherein the adjustment device is adapted to mechanically adjust the restriction device (4, 56), or adapted to hydraulically adjust the restriction device (4, 56) by using hydraulic means which is devoid of hydraulic fluid of the kind having a viscosity that substantially increases when exposed to heat or a magnetic field.

91. An apparatus according to claim 19, wherein the operation device (60) comprises an electrical operation device.

92. An apparatus according to claim 19, wherein the operation device (60) is powered by magnetic energy, non-magnetic energy, electromagnetic energy, non-electromagnetic energy, kinetic energy, non-kinetic energy, thermal energy or non-thermal energy.

93. An apparatus according to claim 1, wherein the control device (6, 10) is activated in a manual or non-manual manner to control the internal source of energy (32, 42, 236) to release energy.

94. An apparatus according to claim 1, further comprising implantable electrical components including at least one voltage level guard.

95. An apparatus according to claim 1, further comprising implantable electrical components including a single voltage level guard.

96. An apparatus according to claim 94 or 95, wherein the electrical components are devoid of any current detector and/or charge level detector.

97. An apparatus according to any of claims 93-96, further comprising an implantable capacitor or accumulator, wherein the charge or discharge of the capacitor or accumulator is controlled by use of the voltage level guard.

98. An apparatus according to claim 14, wherein the released energy comprises electric energy and further comprising an implantable capacitor for producing the train of energy pulses.

99. An apparatus according to claim 97 or 98, wherein the capacitor has a capacity less than 0,1 µF.

100. An apparatus according to claim 14, wherein the wireless energy comprises electromagnetic waves excluding radio waves.

101. An apparatus according to claim 14 or 15, wherein the wireless energy comprises a signal.

102. An apparatus according to claim 1, wherein the implantable energy transforming device (6, 62) is adapted to transform the released wireless energy into energy different from the wireless energy for operation of the restriction device (4, 56).

103. An apparatus according to claim 102, wherein the energy transforming device (6, 62) transforms the wireless energy in the form of sound waves into electric energy for operation of the restriction device (4, 56).

104. An apparatus according to claim 103, wherein the energy transforming device (6, 62) transforms the wireless energy in the form of sound waves directly into electric energy.

105. An apparatus according to claim 103 or 104, wherein the energy transforming device (6, 62) comprises a capacitor.

106. An apparatus according to claim 105, wherein the capacitor is adapted to produce electric pulses from the transformed electric energy.

107. An apparatus according to any of claims 102-106, further comprising an implantable motor or pump (16, 18, 36, 232) for operating the restriction device (4, 56), wherein the motor or pump (16, 18, 36, 232) is powered by the transformed energy.

108. An apparatus according to claim 1, wherein the restriction device (4, 56) is adjustable in a non-manual manner.

109. An apparatus according to any of the preceding claims, wherein the restriction device (4, 56) is embedded in a soft or gel-like material.

110. An apparatus according to any of the preceding claims, wherein the restriction device (4, 56) is embedded in a silicone material having hardness less than 20 Shore.

111. An apparatus according to claim 16, further comprising an activatable source of energy implantable in the patient, wherein the activatable source of energy is activated by wireless energy released from the external source of energy (10, 40), to supply energy which is used in connection with the operation of the restriction device (4, 56).

112. An apparatus according to claim 1, further comprising a switch (238) implantable in the patient for directly or indirectly switching the supply of energy from the internal source of energy (32, 42, 236).

113. The apparatus according to claim 112, wherein the switch (238) directly or indirectly affects the supply of energy from the internal source of energy (32, 42, 236).

114. The apparatus according to claim 112, wherein the switch (238) switches between an "off' mode, in which the internal source of energy (32, 42, 236) is not in use, and an "on" mode, in which the internal source of energy (32, 42, 236) supplies energy for the operation of the restriction device (4, 56).

115. The apparatus according to claim 114, wherein the switch (238) is operable by the wireless energy released from the external source of energy (10, 40).

116. The apparatus according to claim 1, wherein the control device (6, 10) comprises a wireless remote control.

117. The apparatus according to claim 112, wherein the control device (6, 10) comprises a wireless remote control for controlling the internal source of energy (32, 42, 236)

118. The apparatus according to claim 117, wherein the switch (238) is operable by the wireless energy from the external source of energy (10, 40) to switch between an "off" mode, in which the internal source of energy (32, 42, 236) and remote control are not in use, and a "standby" mode, in which the remote control is permitted to control the internal source of energy (32, 42, 236) to supply energy for the operation of the restriction device (4, 56).

119. The apparatus according to claim 113, further comprising an additional internal source of energy implantable in the patient for supplying energy for the operation of the restriction device (4, 56), wherein the switch (238) is operable by energy from the internal source of energy (32, 42, 236) to switch between an "off" mode, in which the additional internal source of energy is not in use, and an "on" mode, in which the additional internal source of energy supplies energy for the operation of the restriction device (4, 56).

120. The apparatus according to claim 119, wherein the control device (6, 10) controls the internal source of energy (32, 42, 236) to operate the switch (238).

121. The apparatus according to claim 120, wherein the control device (6, 10) comprises a wireless remote control.

122. The apparatus according to claim 1, wherein the internal source of energy (32, 42, 236) comprises an electric source of energy.

123. The apparatus according to claim 122, wherein the electric source of energy comprises at least one accumulator, at least one capacitor or at least one rechargeable battery, or a combination of at least one capacitor and at least one rechargeable battery.

124. The apparatus according to claim 122, wherein the electric source of energy comprises an accumulator or a battery having a lifetime of at least 10 years.

125. An apparatus according to claim 1, further comprising a motor (16, 18, 36, 232) implantable in the patient for operating the restriction device (4, 56), wherein the control device (6, 10) is adapted to control the internal source of energy (32, 42, 236) to power the motor (16, 18, 36, 232).

126. An apparatus according to claim 124, wherein the motor (16, 18, 36, 232) comprises a rotary motor, and the control device (6, 10) controls the rotary motor to rotate a desired number of revolutions.

127. An apparatus according to claim 124, wherein the motor (16, 18, 36, 232) comprises a linear motor.

128. An apparatus according to claim 124, wherein the motor (16, 18, 36, 232) comprises a hydraulic or pneumatic fluid motor, and the control device (6, 10) controls the fluid motor.

129. An apparatus according to claim 124, wherein the motor (16, 18, 36, 232) comprises an electric motor having electrically conductive parts made of plastics.

130. An apparatus according to claim 124, wherein the control device (6, 10) is adapted to control the internal source of energy (32, 42, 236) to directly power the motor (16, 18, 36, 232) with the released energy.

131. An apparatus according to claim 124, wherein the control device (6, 10) is adapted to control the external source of energy (10, 40) to release wireless energy in the form of a magnetic field or electromagnetic waves for direct power of the motor (16, 18, 36, 232), as the wireless energy is being released.

132. An apparatus according to claim 124, wherein the internal source of energy (32, 42, 236) comprises at least one accumulator, at least one capacitor or at least one rechargeable battery, or a combination of at least one capacitor and at least one rechargeable battery.

133. An apparatus according to claim 132, wherein the internal source of energy (32, 42, 236) comprises an electric source of energy.

134. An apparatus according to claim 133, wherein the electric source of energy comprises an accumulator, or a battery having a lifetime of at least 10 years.

135. An apparatus according to claim 1, further comprising a pump (16, 18, 36, 232) implantable in the patient for operating the restriction device (4, 56), wherein the control device (6, 10) is adapted to control the internal source of energy (32, 42, 236) to release energy for driving the pump (16, 18, 36, 232).

136. An apparatus according to claim 135, wherein the control device (6, 10) is adapted to control the internal source of energy (32, 42, 236) to directly drive the pump (16, 18, 36, 232) with the released energy.

137. An apparatus according to claim 1, further comprising a pump (16, 18, 36, 232) implantable in the patient for operating the restriction device (4, 56), wherein the control device (6, 10) is adapted to control the external source of energy (10, 40) to release wireless energy in the form of a magnetic field or electromagnetic waves for directly driving the pump (16, 18, 36, 232), as the wireless energy is being released.

138. An apparatus according to claim 135 or 137, wherein the pump (16, 18, 36, 232) is not a plunger type of pump.

## Patentansprüche

1. Vorrichtung zur Behandlung analer Inkontinenz, umfassend:
eine Restriktionseinrichtung (4, 56), die am Kolon (58) oder Rektum angreift, um einen eingeschränkten Fäkaldurchlass in dem Kolon (58) oder Rektum zu bilden, wobei die Restriktionseinrichtung (4, 56) so betätigbar ist, dass sie die Restriktion des Fäkaldurchlasses ändert,
eine interne Energiequelle (32, 42, 236), die in den Patienten implantierbar ist, und
eine Steuerungseinrichtung (6, 10), die von außerhalb des Körpers des Patienten betätigbar ist, um die interne Energiequelle (32, 42, 236) so zu steuern, dass sie Energie zur Verwendung in Verbindung mit der Betätigung der Restriktionseinrichtung (4, 56) freisetzt,
**dadurch gekennzeichnet, dass** die Vorrichtung umfasst:
eine externe Energiequelle (10, 40), die dafür vorgesehen ist, bezüglich des Körpers des Patienten extern zu sein, wenn die Restriktionseinrichtung (4, 56) in diesen implantiert ist, wobei die externe Energiequelle (10, 40) dafür vorgesehen ist, drahtlose Energie freizusetzen, und
eine implantierbare Energieumwandlungseinrichtung (6, 62) zum Umwandeln der freigesetzten drahtlosen Energie in speicherbare Energie,
und wobei
die interne Energiequelle (32, 42, 236) fähig ist, die speicherbare Energie zu speichern, und
die Steuerungseinrichtung (6, 10) dafür vorgesehen ist, die interne Energiequelle (32, 42, 236) so zu steuern, dass sie Energie für die Betätigung der Restriktionseinrichtung (4, 56) liefert, und die Steuerungseinrichtung (6, 10) umfasst:
eine erste Steuerungseinheit (136), die von außerhalb des Körpers des Patienten betätigbar ist, um ein Steuerungssignal zu liefern, und
eine zweite Steuerungseinheit (138), die in den Patienten implantierbar ist und die dafür vorgesehen ist, die Restriktionseinrichtung (4, 56) ansprechend auf das Steuerungssignal zu steuern.

2. Vorrichtung nach Anspruch 1, bei der die zweite Steuerungseinheit (138) programmierbar ist.

3. Vorrichtung nach Anspruch 2, bei der die erste Steuerungseinheit (136) dafür vorgesehen ist, sich außerhalb des Körpers des Patienten zu befinden, wobei die erste Steuerungseinheit (136) durch die zweite Steuerungseinheit (138) programmierbar ist.

4. Vorrichtung nach Anspruch 2, bei der die zweite Steuerungseinheit (138) programmierbar ist, um die Restriktionseinrichtung (4, 56) im Verlauf der Zeit zu steuern.

5. Vorrichtung nach Anspruch 4, bei der die zweite Steuerungseinheit (138) die Restriktionsvorrichtung (4, 56) im Verlauf der Zeit entsprechend einem Aktivititäts-Zeitplanprogramm steuert.

6. Vorrichtung nach Anspruch 4, bei der die zweite Steuerungseinheit (138) einen Mikroprozessor (146) aufweist.

7. Vorrichtung nach Anspruch 3, bei der die erste Steuerungseinheit (136) die zweite Steuerungseinheit (138) mit Daten entsprechend einem Lademodus lädt, der nur für einen Arzt/eine Ärztin zugelassen ist.

8. Vorrichtung nach Anspruch 3, bei der die erste Steuerungseinheit (136) die zweite Steuerungseinheit (138) entsprechend einem Arztmodus steuert, der nur für einen Arzt/eine Ärztin zugelassen ist.

9. Vorrichtung nach Anspruch 3, bei der die erste Steuerungseinheit (136) die zweite Steuerungseinheit (138) entsprechend einem Patientenmodus steuert, der für den Patienten zugelassen ist.

10. Vorrichtung nach Anspruch 1, bei der die interne Energiequelle (32, 42, 236) einen Akkumulator (28) aufweist.

11. Vorrichtung nach Anspruch 10, bei der der Akkumulator (28) einen elektrischen Akkumulator aufweist.

12. Vorrichtung nach Anspruch 11, bei der der elektrische Akkumulator wenigstens einen Kondensator oder wenigstens eine wiederaufladbare Batterie oder eine Kombination aus wenigstens einem Kondensator und wenigstens einer wiederaufladbaren Batterie aufweist.

13. Vorrichtung nach Anspruch 1, die weiterhin eine Batterie aufweist, die in den Patienten implantierbar ist, um implantierbaren, elektrische Energie benötigenden Bauteilen der Vorrichtung elektrische Energie zuzuführen.

14. Vorrichtung nach Anspruch 1, bei der die Steuerungseinrichtung (6, 10) dafür vorgesehen ist, die externe Energiequelle (10, 40) so zu steuern, dass sie drahtlose Energie für eine direkte Verwendung im Zusammenhang mit der Betätigung der Restriktionseinrichtung (4, 56) freisetzt.

15. Vorrichtung nach Anspruch 14, bei der die Steuerungseinrichtung (6, 10) dafür vorgesehen ist, die externe Energiequelle (10, 40) so zu steuern, dass sie in Intervallen drahtlose Energie in Form einer Energieimpulsfolge für eine direkte Verwendung im Zusammenhang mit der Betätigung der Restriktionseinrichtung (4, 56) freisetzt.

16. Vorrichtung nach Anspruch 14 oder 15, bei der die Restriktionseinrichtung (6, 10) auf nicht magnetische, nicht thermische oder nicht mechanische Art durch Verwendung der freigesetzten drahtlosen Energie betätigbar ist.

17. Vorrichtung nach Anspruch 1, bei der die externe Energiequelle (10, 40) eine nukleare Energiequelle umfasst.

18. Vorrichtung nach Anspruch 1, bei der die externe Energiequelle (10, 40) eine chemische Energiequelle umfasst.

19. Vorrichtung nach Anspruch 1, die weiterhin eine in den Patienten implantierbare Betätigungseinrichtung (60) zum Betätigen der Restriktionseinrichtung (4, 56) umfasst.

20. Vorrichtung nach Anspruch 19, bei der die drahtlose Energie direkt oder indirekt die Betätigungseinrichtung (60) antreibt.

21. Vorrichtung nach Anspruch 19 oder 20, bei der die Steuerungseinrichtung (6, 10) die Betätigungseinrichtung (60) so steuert, dass sie die Restriktionseinrichtung (4, 56) betätigt.

22. Vorrichtung nach Anspruch 21, bei der die Betätigungseinrichtung (60) ein hydraulisches Mittel und wenigstens ein Ventil zur Steuerung eines Fluidstroms in dem hydraulischen Mittel aufweist.

23. Vorrichtung nach Anspruch 22, bei der die Steuerungseinrichtung (6, 10) eine drahtlose Fernsteuerung zur Steuerung des Ventils aufweist.

24. Vorrichtung nach Anspruch 21, bei der die Restriktionseinrichtung (4, 56) ein hydraulisches Mittel umfasst und die Betätigungseinrichtung (60) einen Behälter (20, 34, 46, 230) umfasst, der eine Fluidkammer mit variablem Volumen bildet, die mit dem hydraulischen Mittel verbunden ist, und die Betätigungseinrichtung (60) dafür vorgesehen ist, Fluid von der Kammer zu dem hydraulischen Mittel zu verteilen, indem das Volumen der Kammer reduziert wird, und Fluid von dem hydraulischen Mittel zu der Kammer abzuziehen, indem das Volumen der Kammer erhöht wird.

25. Vorrichtung nach Anspruch 19 oder 20, bei der die Betätigungseinrichtung (60) einen Motor (16, 18, 36, 232) umfasst.

26. Vorrichtung nach Anspruch 25, bei der der Motor (16, 18, 36, 232) einen Drehmotor umfasst und die Steuerungseinrichtung (6, 10) den Drehmotor so steuert, dass er eine gewünschte Zahl von Umdrehungen ausführt.

27. Vorrichtung nach Anspruch 25, bei der der Motor (16, 18, 36, 232) einen Linearmotor umfasst.

28. Vorrichtung nach Anspruch 25, bei der der Motor (16, 18, 36, 232) einen hydraulischen oder pneumatischen Fluidmotor umfasst und die Steuerungseinrichtung (6, 10) den Fluidmotor steuert.

29. Vorrichtung nach Anspruch 25, bei der der Motor (16, 18, 36, 232) einen Elektromotor mit aus Kunststoff hergestellten elektrisch leitenden Teilen umfasst.

30. Vorrichtung nach einem der Ansprüche 19-21, bei der die Steuerungseinrichtung (6, 10) polarisierte Energie von der internen Energiequelle (32, 42, 236) freisetzt.

31. Vorrichtung nach einem der Ansprüche 19-21, bei der die Steuerungseinrichtung (6, 10) die Polarität der freigesetzten Energie verschiebt, um die Betätigungseinrichtung (60) umzukehren.

32. Vorrichtung nach einem der Ansprüche 19-21, bei der die Betätigungseinrichtung (60) einen Elektromotor (232) umfasst und die freigesetzte Energie elektrische Energie umfasst.

33. Vorrichtung nach einem der Anspruche 19-21 und 25, bei der die Restriktionseinrichtung (4, 56) so betätigbar ist, dass sie eine umkehrbare Funktion ausführt.

34. Vorrichtung nach Anspruch 33, die weiterhin eine in den Patienten implantierbare Umkehreinrichtung (14, 38, 52, 234) umfasst, um die von der Restriktionseinrichtung (4, 56) ausgeführte Funktion umzukehren.

35. Vorrichtung nach Anspruch 34, bei der die Steuerungseinrichtung (6, 10) die Umkehreinrichtung (14, 38, 52, 234) steuert, um die von der Restriktionseinrichtung (4, 56) ausgeführte Funktion umzukehren.

36. Vorrichtung nach Anspruch 34, bei der die Umkehreinrichtung (14, 38, 52, 234) ein hydraulisches Mittel umfasst, das ein Ventil (38) aufweist, um die Strömungsrichtung eines Fluids in dem hydraulischen Mittel zu verschieben.

37. Vorrichtung nach Anspruch 36, bei der die Umkehreinrichtung (14, 38, 52, 234) eine mechanische Umkehreinrichtung (14, 52) umfasst.

38. Vorrichtung nach Anspruch 37, bei der die mechanische Umkehreinrichtung einen Schalter (14) aufweist.

39. Vorrichtung nach Anspruch 37, bei der die Umkehreinrichtung (14, 38, 52, 234) ein Getriebe (52) umfasst.

40. Vorrichtung nach Anspruch 34, bei der die Umkehreinrichtung (14, 38, 52, 234) einen Schalter (14) aufweist.

41. Vorrichtung nach Anspruch 40, bei der der Schalter (14) der Umkehreinrichtung von der freigesetzten Energie betätigbar ist.

42. Vorrichtung nach Anspruch 41, bei der die Steuerungseinrichtung (6, 10) die Betätigung des Schalters (14) der Umkehreinrichtung steuert, indem die Polarität der dem Schalter (14) zugeführten freigesetzten Energie verschoben wird.

43. Vorrichtung nach Anspruch 40, bei der der Schalter (14) einen elektrischen Schalter umfasst und die interne Energiequelle (32, 42, 236) elektrische Energie für die Betätigung des Schalters (14) liefert.

44. Vorrichtung nach Anspruch 40, bei der die Betätigungseinrichtung (60) einen Motor (16, 18, 36, 232) umfasst und die Umkehreinrichtung (14, 38, 52, 234) den Motor (16, 18, 36, 232) umkehrt.

45. Vorrichtung nach einem der Ansprüche 1, 19-21, bei der die Restriktionseinrichtung (4, 56) ein hydraulisches Mittel umfasst und die Betätigungseinrichtung (60) dafür vorgesehen ist, ein hydraulisches Fluid in dem hydraulischen Mittel zu leiten.

46. Vorrichtung nach Anspruch 45, bei der die Betätigungseinrichtung (60) einen Motor (16, 18, 36, 232) umfasst.

47. Vorrichtung nach Anspruch 45 oder 46, bei der die Betätigungseinrichtung (60) eine Fluidleitung, die mit dem hydraulischen Mittel der Restriktionseinrichtung (4, 56) verbunden ist, und einen Behälter (20, 34, 46, 230) für Fluid aufweist, wobei der Behälter (20, 34, 46, 230) einen Teil der Leitung bildet.

48. Vorrichtung nach Anspruch 47, bei der das hydraulische Mittel und die Leitung kein Rückschlagventil aufweisen.

49. Vorrichtung nach Anspruch 48, bei der der Behälter (20, 34, 46, 230) eine Fluidkammer mit variablem Volumen bildet und die Betätigungseinrichtung (60) dafür vorgesehen ist, Fluid von der Kammer an das hydraulische Mittel der Restriktionseinrichtung (4, 56) zu verteilen, indem das Volumen der Kammer reduziert wird, und Fluid von dem hydraulischen Mittel zu der Kammer abzuziehen, indem das Volumen der Kammer erhöht wird.

50. Vorrichtung nach einem der vorigen Ansprüche, die weiterhin wenigstens einen implantierbaren Sensor (54) zum Erfassen von wenigstens einem physischen Parameter des Patienten umfasst.

51. Vorrichtung nach Anspruch 50, bei der der Sensor (54) einen Drucksensor zum direkten oder indirekten Erfassen des Drucks in dem Kolon (58) oder Rektum als physischem Parameter umfasst.

52. Vorrichtung nach Anspruch 50 oder 51, bei der die Steuerungseinrichtung (6, 10) die Restriktionseinrichtung (4, 56) ansprechend auf Signale von dem Sensor (54) steuert.

53. Vorrichtung nach Anspruch 52, bei der die zweite Steuerungseinheit (138) die Restriktionseinrichtung (4, 56) ansprechend auf Signale von dem Sensor (54) steuert.

54. Vorrichtung nach Anspruch 53, bei der die erste Steuerungseinheit (136) die Restriktionseinrichtung (4, 56) ansprechend auf Signale von dem Sensor (54) steuert.

55. Vorrichtung nach Anspruch 54, bei der die erste Steuerungseinheit (136) Informationen über den physischen Parameter speichert, der von dem Sensor (54) erfasst wird, und manuell betätigt wird, um die Restriktionseinrichtung (4, 56) auf der Grundlage der gespeicherten Informationen zu steuern.

56. Vorrichtung nach einem der Ansprüche 50-55, die weiterhin wenigstens einen implantierbaren Sender zum Senden von Informationen über den von dem Sensor (54) erfassten physischen Parameter umfasst.

57. Vorrichtung nach einem der vorigen Ansprüche, die weiterhin eine externe Datenkommunikationseinrichtung, die dafür vorgesehen ist, sich außerhalb des Körpers des Patienten zu befinden, sowie eine interne Datenkommunikationseinrichtung umfasst, die in den Körper des Patienten implantierbar ist, um mit der externen Kommunikationseinrichtung zu kommunizieren, wobei die interne Datenkommunikationseinrichtung patientenbezogene Daten zurück an die externe Datenkommunikationseinrichtung überträgt oder die externe Datenkommunikationseinrichtung Daten an die interne Datenkommunikationseinrichtung überträgt.

58. Vorrichtung nach Anspruch 57, bei der die interne Datenkommunikationseinrichtung Daten überträgt, die sich auf die Restriktionseinrichtung (4, 56) beziehen.

59. Vorrichtung nach Anspruch 57 oder 58, bei der die implantierbare Kommunikationseinrichtung Daten überträgt, die sich auf wenigstens ein physisches Signal des Patienten beziehen.

60. Vorrichtung nach einem der vorigen Ansprüche, bei der die Restriktionseinrichtung (4, 56) dafür vorgesehen ist, die Restriktion des Fäkaldurchlasses zu steuern.

61. Vorrichtung nach Anspruch 60, bei der die Restriktionseinrichtung (4, 56) so betätigbar ist, dass sie den Fäkaldurchlass vergrößert und einschränkt, wenn sie in den Patienten implantiert ist.

62. Vorrichtung nach Anspruch 60 oder 61, bei der die Restriktionseinrichtung (4, 56) dafür vorgesehen ist, stufenlos die Restriktion des Fäkaldurchlasses zu steuern, wenn sie in den Patienten implantiert ist.

63. Vorrichtung nach einem der vorigen Ansprüche, bei der die Steuerungseinrichtung (6, 10) dafür vorgesehen ist, die externe Energiequelle (10, 40) so zu steuern, dass sie drahtlose elektrische Energie freisetzt, und bei der die Energieumwandlungseinrichtung (6, 62) dafür vorgesehen ist, die elektrische Energie in kinetische Energie für die Betätigung der Restriktionseinrichtung (4, 56) umzuwandeln.

64. Vorrichtung nach Anspruch 63, bei der die Restriktionseinrichtung (4, 56) direkt mit der kinetischen Energie betätigt wird, wenn die Energieumwandlungseinrichtung (6, 62) die elektrische Energie in die kinetische Energie umwandelt.

65. Vorrichtung nach einem der vorigen Ansprüche, bei der die Steuerungseinrichtung (6, 10) die interne Energiequelle (32, 42, 236) so steuert, dass sie magnetische Energie, nicht magnetische Energie, elektromagnetische Energie, nicht elektromagnetische Energie, kinetische Energie, nicht kinetische Energie, Schallenergie, Nicht-Schallenergie, Wärmeenergie oder Nicht-Wärmeenergie freisetzt.

66. Vorrichtung nach einem der vorigen Ansprüche, bei der die Restriktionseinrichtung (4, 56) nicht aufblasbar ist.

67. Vorrichtung nach einem der vorigen Ansprüche, bei der die Steuerungseinrichtung (6, 10) die interne Energiequelle (32, 42, 236) so steuert, dass sie eine bestimmte Zeit lang Energie freisetzt.

68. Vorrichtung nach einem der Ansprüche 1-66, bei der die Steuerungseinrichtung (6, 10) die externe Energiequelle (10, 40) so steuert, dass sie Energie in einer bestimmten Zahl von Energieimpulsen freisetzt.

69. Vorrichtung nach einem der vorigen Ansprüche, bei der die Steuerungseinrichtung (6, 10) dafür vorgesehen ist, die interne Energiequelle (32, 42, 236) so zu steuern, dass sie Energie auf nicht invasive Art freisetzt.

70. Vorrichtung nach einem der vorigen Ansprüche, bei der die Steuerungseinrichtung (6, 10) eine drahtlose Fernsteuerung umfasst, um wenigstens ein drahtloses Steuerungssignal zur Steuerung der Restriktionseinrichtung (4, 56) zu übertragen.

71. Vorrichtung nach Anspruch 70, bei der die Fernsteuerung in der Lage ist, Informationen über den Zustand der Restriktionseinrichtung (4, 56) zu erlangen, wenn die Restriktionseinrichtung (4, 56) implantierbar ist, und die Restriktionseinrichtung (4, 56) ansprechend auf die Informationen zu steuern.

72. Vorrichtung nach Anspruch 70 oder 71, bei der die drahtlose Fernsteuerung wenigstens einen externen Signaltransmitter oder -transceiver und wenigstens einen internen Signalempfänger oder -transceiver umfasst, der in den Patienten implantierbar ist.

73. Vorrichtung nach Anspruch 70 oder 71, bei der die drahtlose Fernsteuerung wenigstens einen externen Signalempfänger oder -transceiver und wenigstens einen internen Signaltransmitter oder -transceiver umfasst, der in den Patienten implantierbar ist.

74. Vorrichtung nach einem der Ansprüche 70-73, bei der die Fernsteuerung in der Lage ist, Informationen bezüglich der Restriktionseinrichtung (4, 56) vom Inneren des Körpers des Patienten zu dessen Außenseite zu senden.

75. Vorrichtung nach Anspruch 74, bei der die Fernsteuerung die Restriktionseinrichtung (4, 56) ansprechend auf die Informationen steuert.

76. Vorrichtung nach einem der Ansprüche 70-75, bei der die Fernsteuerung ein Trägersignal zum Tragen des Steuerungssignals überträgt.

77. Vorrichtung nach Anspruch 76, bei der das Trägersignal frequenz-, amplituden- oder frequenz- und amplitudenmoduliert ist.

78. Vorrichtung nach Anspruch 76 oder 77, bei der das Trägersignal digital, analog oder digital und analog ist.

79. Vorrichtung nach einem der Ansprüche 76-78, bei der das mit dem Trägersignal verwendete Steuerungssignal frequenz-, amplituden- oder frequenz- und amplitudenmoduliert ist.

80. Vorrichtung nach einem der Ansprüche 70-79, bei der das Steuerungssignal ein Wellensignal umfasst, das eines der folgenden umfasst: ein Schallwellensignal, das ein Ultraschallwellensignal umfasst, ein elektromagnetisches Wellensignal, das ein Infrarotlichtsignal, ein Signal aus sichtbarem Licht, ein ultraviolettes Lichtsignal und ein Laserlichtsignal umfasst, ein Mikrowellensignal, ein Radiowellensignal, ein Röntgenstrahlungssignal und ein Gammastrahlungssignal.

81. Vorrichtung nach einem der Ansprüche 70-79, bei der das Steuerungssignal ein elektrisches, magnetisches oder elektrisches und magnetisches Feld umfasst.

82. Vorrichtung nach einem der Ansprüche 70-80, bei der das Steuerungssignal digital, analog oder digital und analog ist.

83. Vorrichtung nach Anspruch 82, bei der die Fernsteuerung ein elektromagnetisches Trägerwellensignal zum Tragen des digitalen oder analogen Steuerungssignals überträgt.

84. Vorrichtung nach einem der Ansprüche 70-83, bei der das Steuerungssignal in Impulsen durch die drahtlose Fernsteuerung übertragen wird.

85. Vorrichtung nach einem der Ansprüche 14-16 oder 61-63, das weiterhin einen implantierbaren Stabilisator zur Stabilisierung der Energie umfasst, die von der Steuerungseinrichtung (6, 10) freigesetzt wird.

86. Vorrichtung nach Anspruch 85, bei der die von der Steuerungseinrichtung (6, 10) freigesetzte Energie elektrische Energie umfasst und der Stabilisator wenigstens einen Kondensator (162) umfasst.

87. Vorrichtung nach Anspruch 1, bei der die Restriktionseinrichtung (4, 56) von der freigesetzten Energie auf manuelle, mechanische, thermische oder magnetische Art betätigbar ist.

88. Vorrichtung nach Anspruch 1, bei der die Restriktionseinrichtung (4, 56) von der freigesetzten Energie auf nicht manuelle, nicht mechanische, nicht thermische oder nicht magnetische Art betätigbar ist.

89. Vorrichtung nach Anspruch 14 oder 16, bei der die Steuerungseinrichtung (6, 10) dafür vorgesehen ist, die interne Energiequelle (32, 42, 236) so zu steuern, dass sie elektrische Energie freisetzt, und die weiterhin einen implantierbaren Kondensator aufweist, um die Energieimpulsfolge aus der freigesetzten Energie zu erzeugen.

90. Vorrichtung nach Anspruch 1, die weiterhin eine Einstelleinrichtung zum Einstellen der Restriktionseinrichtung (4, 56) aufweist, um den Restriktions-Fäkaldurchlass zu ändern, wobei die Einstelleinrichtung dafür vorgesehen ist, die Restriktionseinrichtung (4, 56) mechanisch einzustellen, oder dafür vorgesehen ist, die Restriktionseinrichtung (4, 56) hydraulisch einzustellen, indem ein hydraulisches Mittel verwendet wird, das kein hydraulisches Fluid der Art aufweist, das eine Viskosität hat, die sich wesentlich erhöht, wenn es Wärme oder einem Magnetfeld ausgesetzt wird.

91. Vorrichtung nach Anspruch 19, bei der die Betätigungseinrichtung (60) eine elektrische Betätigungseinrichtung aufweist.

92. Vorrichtung nach Anspruch 19, bei der die Betätigungseinrichtung (60) durch magnetische Energie, nicht magnetische Energie, elektromagnetische Energie, nicht elektromagnetische Energie, kinetische Energie, nicht kinetische Energie, Wärmeenergie oder Nicht-Wärmeenergie angetrieben wird.

93. Vorrichtung nach Anspruch 1, bei der die Steuerungseinrichtung (6, 10) auf manuelle oder nicht manuelle Art aktiviert wird, um die interne Energiequelle (32, 42, 236) so zu steuern, dass sie Energie freisetzt.

94. Vorrichtung nach Anspruch 1, die weiterhin implantierbare elektrische Bauteile aufweist, die wenigstens einen Spannungshöhenbegrenzer umfassen.

95. Vorrichtung nach Anspruch 1, die weiterhin implantierbare elektrische Bauteile aufweist, die einen Einzel-Spannungshöhenbegrenzer umfassen.

96. Vorrichtung nach einem der Ansprüche 94 oder 95, bei der die elektrischen Bauteile keine Stromstärkenerfassungseinrichtung und/oder Ladungshöhenerfassungseinrichtung aufweisen.

97. Vorrichtung nach einem der Ansprüche 93-96, die weiterhin einen implantierbaren Kondensator oder Akkumulator aufweist, wobei die Ladung oder Entladung des Kondensators oder Akkumulators durch Verwendung des Spannungshöhenbegrenzers gesteuert wird.

98. Vorrichtung nach Anspruch 14, bei der die freigesetzte Energie elektrische Energie aufweist, und die weiterhin einen implantierbaren Kondensator zur Erzeugung der Energieimpulsfolge umfasst.

99. Vorrichtung nach Anspruch 97 oder 98, bei der der Kondensator eine Kapazität von weniger als 0,1 µF hat.

100. Vorrichtung nach Anspruch 14, bei der die drahtlose Energie elektromagnetische Wellen außer Radiowellen umfasst.

101. Vorrichtung nach Anspruch 14 oder 15, bei der die drahtlose Energie ein Signal umfasst.

102. Vorrichtung nach Anspruch 1, bei der die implantierbare Energieumwandlungseinrichtung (6, 62) dafür vorgesehen ist, die freigesetzte drahtlose Energie in Energie umzuwandeln, die sich von der drahtlosen Energie für die Betätigung der Restriktionseinrichtung (4, 56) unterscheidet.

103. Vorrichtung nach Anspruch 102, bei der die Energieumwandlungseinrichtung (6, 62) die drahtlose Energie in Form von Schallwellen in elektrische Energie für die Betätigung der Restriktionseinrichtung (4, 56) umwandelt.

104. Vorrichtung nach Anspruch 103, bei der die Energieumwandlungseinrichtung (6, 62) die drahtlose Energie in Form von Schallwellen direkt in elektrische Energie umwandelt.

105. Vorrichtung nach Anspruch 103 oder 104, bei der die Energieumwandlungseinrichtung (6, 62) einen Kondensator umfasst.

106. Vorrichtung nach Anspruch 105, bei der der Kondensator dafür vorgesehen ist, elektrische Impulse aus der umgewandelten elektrischen Energie zu erzeugen.

107. Vorrichtung nach einem der Ansprüche 102-106, die weiterhin einen implantierbaren Motor oder eine implantierbare Pumpe (16, 18, 36, 232) zum Betätigen der Restriktionseinrichtung (4, 56) umfasst, wobei der Motor oder die Pumpe (16, 18, 36, 232) von der umgewandelten Energie angetrieben wird.

108. Vorrichtung nach Anspruch 1, bei der die Restriktionseinrichtung (4, 56) auf nicht manuelle Art einstellbar ist.

109. Vorrichtung nach einem der vorigen Ansprüche, bei der die Restriktionseinrichtung (4, 56) in einem weichen oder gelartigen Material eingebettet ist.

110. Vorrichtung nach einem der vorigen Ansprüche, bei der die Restriktionseinrichtung (4, 56) in einem Silikonmaterial mit einer Shore-Härte von weniger als 20 eingebettet ist.

111. Vorrichtung nach Anspruch 16, die weiterhin eine aktivierbare Energiequelle umfasst, die in den Patienten implantierbar ist, wobei die aktivierbare Energiequelle durch drahtlose Energie aktiviert wird, die von der externen Energiequelle (10, 40) freigesetzt wird, um Energie zu liefern, die im Zusammenhang mit der Betätigung der Restriktionseinrichtung (4, 56) verwendet wird.

112. Vorrichtung nach Anspruch 1, die weiterhin einen in den Patienten implantierbaren Schalter (238) aufweist, um direkt oder indirekt die Energiezufuhr von der internen Energiequelle (32, 42, 236) umzuschalten.

113. Vorrichtung nach Anspruch 112, bei der der Schalter (238) direkt oder indirekt die Energiezufuhr von der internen Energiequelle (32, 42, 236) beeinflusst.

114. Vorrichtung nach Anspruch 112, bei der der Schalter (238) zwischen einem "Aus"-Modus, bei dem die interne Energiequelle (32, 42, 236) nicht in Betrieb ist, und einem "Ein"-Modus, bei dem die interne Energiequelle (32, 42, 236) Energie für die Betätigung der Restriktionseinrichtung (4, 56) liefert, umschaltet.

115. Vorrichtung nach Anspruch 114, bei der der Schalter (238) durch die drahtlose Energie betätigbar ist, die von der externen Energiequelle (10, 40) freigesetzt wird.

116. Vorrichtung nach Anspruch 1, bei der die Steuerungseinrichtung (6, 10) eine drahtlose Fernsteuerung umfasst.

117. Vorrichtung nach Anspruch 112, bei der die Steuerungseinrichtung (6, 10) eine drahtlose Fernsteuerung zur Steuerung der internen Energiequelle (32, 42, 236) aufweist.

118. Vorrichtung nach Anspruch 117, bei der der Schalter (238) durch die drahtlose Energie von der externen Energiequelle (10, 40) so betätigbar ist, dass er zwischen einem "Aus"-Modus, bei dem die interne Energiequelle (32, 42, 236) und die Fernsteuerung nicht in Betrieb sind, und einem "Standby"-Modus, bei dem die Fernsteuerung die interne Energiequelle (32, 42, 236) so steuern kann, dass sie Energie für die Betätigung der Restriktionseinrichtung (4, 56) liefert, umschaltet.

119. Vorrichtung nach Anspruch 113, die weiterhin eine zusätzliche interne Energiequelle aufweist, die in den Patienten implantierbar ist, um Energie für die Betätigung der Restriktionseinrichtung (4, 56) zu liefern, wobei der Schalter (238) durch Energie von der internen Energiequelle (32, 42, 236) so betätigbar ist, dass er zwischen einem "Aus"-Modus, bei der die zusätzliche interne Energiequelle nicht verwendet wird, und einem "Ein"-Modus, bei dem die zusätzliche interne Energiequelle Energie für die Betätigung der Restriktionseinrichtung (4, 56) liefert, umschaltet.

120. Vorrichtung nach Anspruch 119, bei der die Steuerungseinrichtung (6, 10) die interne Energiequelle (32, 42, 236) so steuert, dass sie den Schalter (238) betätigt.

121. Vorrichtung nach Anspruch 120, bei der die Steuerungseinrichtung (6, 10) eine drahtlose Fernsteuerung aufweist.

122. Vorrichtung nach Anspruch 1, bei der die interne Energiequelle (32, 42, 236) eine elektrische Energiequelle umfasst.

123. Vorrichtung nach Anspruch 122, bei der die elektrische Energiequelle wenigstens einen Akkumulator, wenigstens einen Kondensator oder wenigstens eine wiederaufladbare Batterie oder eine Kombination von wenigstens einem Kondensator und wenigstens einer wiederaufladbaren Batterie umfasst.

124. Vorrichtung nach Anspruch 122, bei der die elektrische Energiequelle einen Akkumulator oder eine Batterie umfasst, deren Lebensdauer wenigstens 10 Jahre beträgt.

125. Vorrichtung nach Anspruch 1, die weiterhin einen in den Patienten implantierbaren Motor (16, 18, 36, 232) zur Steuerung der Restriktionseinrichtung (4, 56) aufweist, wobei die Steuerungseinrichtung (6, 10) dafür vorgesehen ist, die interne Energiequelle (32, 42, 236) zu steuern, um den Motor (16, 18, 36, 232) anzutreiben.

126. Vorrichtung nach Anspruch 124, bei der der Motor (16, 18, 36, 232) einen Drehmotor umfasst und die Steuerungseinrichtung (6, 10) den Drehmotor so steuert, dass er eine gewünschte Zahl von Umdrehungen durchführt.

127. Vorrichtung nach Anspruch 124, bei der der Motor (16, 18, 36, 232) einen Linearmotor umfasst.

128. Vorrichtung nach Anspruch 124, bei der der Motor (16, 18, 36, 232) einen hydraulischen oder pneumatischen Fluidmotor umfasst und die Steuerungseinrichtung (6, 10) den Fluidmotor steuert.

129. Vorrichtung nach Anspruch 124, bei der der Motor (16, 18, 36, 232) einen Elektromotor umfasst, der aus Kunststoff hergestellte elektrisch leitfähige Teile aufweist.

130. Vorrichtung nach Anspruch 124, bei der die Steuerungseinrichtung (6, 10) dafür vorgesehen ist, die interne Energiequelle (32, 42, 236) so zu steuern, dass sie den Motor (16, 18, 36, 232) mit der freigesetzten Energie antreibt.

131. Vorrichtung nach Anspruch 124, bei der die Steuerungseinrichtung (6, 10) dafür vorgesehen ist, die externe Energiequelle (10, 40) so zu steuern, dass sie drahtlose Energie in Form eines magnetischen Felds oder elektromagnetischer Wellen für einen direkten Antrieb des Motors (16, 18, 36, 232) freisetzt, wenn die drahtlose Energie freigesetzt wird.

132. Vorrichtung nach Anspruch 124, bei der die interne Energiequelle (32, 42, 236) wenigstens einen Akkumulator, wenigstens einen Kondensator oder wenigstens eine wiederaufladbare Batterie oder eine Kombination aus wenigstens einem Kondensator und wenigstens einer wiederaufladbaren Batterie umfasst.

133. Vorrichtung nach Anspruch 132, bei der die interne Energiequelle (32, 42, 236) eine elektrische Energiequelle umfasst.

134. Vorrichtung nach Anspruch 133, bei der die elektrische Energiequelle einen Akkumulator oder eine Batterie mit einer Lebensdauer von wenigstens 10 Jahren umfasst.

135. Vorrichtung nach Anspruch 1, die weiterhin eine in den Patienten implantierbare Pumpe (16, 18, 36, 232) zur Betätigung der Restriktionseinrichtung (4, 56) umfasst, wobei die Steuerungseinrichtung (6, 10) dafür vorgesehen ist, die interne Energiequelle (32, 42, 236) so zu steuern, dass sie Energie zum Antrieb der Pumpe (16, 18, 36, 232) freisetzt.

136. Vorrichtung nach Anspruch 135, bei der die Steuerungseinrichtung (6, 10) dafür vorgesehen ist, die interne Energiequelle (32, 42, 236) so zu steuern, dass die Pumpe (16, 18, 36, 232) direkt mit der freigesetzten Energie angetrieben wird.

137. Vorrichtung nach Anspruch 1, die weiterhin eine in den Patienten implantierbare Pumpe (16, 18, 36, 232) zur Betätigung der Restriktionseinrichtung (4, 56) umfasst, wobei die Steuerungseinrichtung (6, 10) dafür vorgesehen ist, die externe Energiequelle (10, 40) so zu steuern, dass sie drahtlose Energie in Form eines magnetischen Felds oder elektromagnetischer Wellen für einen direkten Antrieb der Pumpe (16, 18, 36, 232) freisetzt, wenn die drahtlose Energie freigesetzt wird.

138. Vorrichtung nach Anspruch 135 oder 137, bei der die Pumpe (16, 18, 36, 232) kein Plunger-Pumpentyp ist.

## Revendications

1. Appareil pour le traitement de l'incontinence anale, comprenant :
un dispositif de restriction (4, 56) destiné à être mis au contact du côlon (58) ou du rectum pour former une voie de passage restreinte des matières fécales dans le côlon (58) ou le rectum, le dispositif de restriction (4, 56) pouvant être commandé de façon à changer la restriction de la voie de passage des matières fécales,
une source d'énergie interne (32, 42, 236) implantable dans le patient, et
un dispositif de contrôle (6, 10) pouvant être commandé de l'extérieur du corps du patient pour contrôler la source d'énergie interne (32, 42, 236) de façon à libérer de l'énergie utilisée en relation avec la commande du dispositif de restriction (4, 56),
**caractérisé en ce que** l'appareil comprend :
une source externe d'énergie (10, 40) destinée à être à l'extérieur du corps du patient lorsque le dispositif de restriction (4, 56) est implanté dans celui-ci, la source d'énergie extérieure (10, 40) étant adaptée pour libérer de l'énergie sans fil, et
un dispositif implantable de transformation d'énergie (6, 62) pour transformer l'énergie sans fil libérée en énergie stockable, et dans lequel
la source d'énergie interne (32, 42, 236) peut stocker l'énergie stockable, et
le dispositif de contrôle (6, 10) est adapté de façon à contrôler la source d'énergie interne (32, 42, 236) pour fournir de l'énergie pour la commande du dispositif de restriction (4, 56) et le dispositif de contrôle comprend :
une première unité de contrôle (136) commandable de l'extérieur du corps du patient de façon à fournir un signal de contrôle, et
une deuxième unité de contrôle (138) pouvant être implantée dans le patient, adaptée pour contrôler le dispositif de restriction (4, 56) en réponse au signal de contrôle.

2. Appareil selon la revendication 1, dans lequel la deuxième unité de contrôle (138) est programmable.

3. Appareil selon la revendication 2, dans lequel la première unité de contrôle (136) est destinée à être à l'extérieur du corps du patient, la première unité de contrôle (136) pouvant être programmée par la deuxième unité de contrôle (138).

4. Appareil selon la revendication 2, dans lequel la deuxième unité de contrôle (138) peut être programmée pour contrôler le dispositif de restriction (4, 56) dans le temps.

5. Appareil selon la revendication 4, dans lequel la deuxième unité de contrôle (138) contrôle le dispositif de restriction (4, 56) dans le temps, selon un programme d'activité.

6. Appareil selon la revendication 4, dans lequel la deuxième unité de contrôle (138) comprend un microprocesseur (146).

7. Appareil selon la revendication 3, dans lequel la première unité de contrôle (136) charge la deuxième unité de contrôle (138) avec des données selon un mode de chargement autorisé uniquement pour un médecin.

8. Appareil selon la revendication 3, dans lequel la première unité de contrôle (136) contrôle la deuxième unité de contrôle (138) selon un mode médical autorisé uniquement pour un médecin.

9. Appareil selon la revendication 3, dans lequel la première unité de contrôle (136) contrôle la deuxième unité de contrôle (138) selon un mode patient autorisé pour le patient.

10. Appareil selon la revendication 1, dans lequel la source d'énergie interne (32, 42, 236) comprend un accumulateur (28).

11. Appareil selon la revendication 10, dans lequel l'accumulateur comprend un accumulateur électrique.

12. Appareil selon la revendication 11, dans lequel l'accumulateur électrique comprend au moins un condensateur ou au moins une batterie rechargeable, ou une combinaison d'au moins un condensateur et d'au moins une batterie rechargeable.

13. Appareil selon la revendication 1, comprenant en outre une batterie implantable dans le patient pour fournir une énergie électrique à des éléments de l'appareil consommant de l'énergie électrique.

14. Appareil selon la revendication 1, dans lequel le dispositif de contrôle (6, 10) est adapté de façon à contrôler la source d'énergie externe (10, 40) pour libérer de l'énergie sans fil pour utilisation directe en relation avec la commande du dispositif de restriction (4, 56).

15. Appareil selon la revendication 14, dans lequel le dispositif de contrôle (6, 10) est adapté de façon à contrôler la source d'énergie externe (10, 40) pour libérer de manière intermittente de l'énergie sans fil sous la forme d'une série d'impulsions d'énergie pour utilisation directe en relation avec la commande du dispositif de restriction (4, 56).

16. Appareil selon la revendication 14 ou 15, dans lequel le dispositif de restriction (6, 10) est commandable de manière non magnétique, non thermique ou non mécanique en utilisant ladite énergie libérée sans fil.

17. Appareil selon la revendication 1, dans lequel la source d'énergie externe (10, 40) comprend une source d'énergie nucléaire.

18. Appareil selon la revendication 1, dans lequel la source d'énergie externe (10, 40) comprend une source d'énergie chimique.

19. Appareil selon la revendication 1, comprenant en outre un dispositif de commande (60) implantable dans le patient pour commander le dispositif de restriction (4, 56).

20. Appareil selon la revendication 19, dans lequel l'énergie sans fil alimente directement ou indirectement le dispositif de commande (60).

21. Appareil selon la revendication 19 ou 20, dans lequel le dispositif de contrôle (6, 10) contrôle le dispositif commande (60) de façon à commander le dispositif de restriction (4, 56).

22. Appareil selon la revendication 21, dans lequel dispositif de commande (60) comprend un moyen hydraulique et au moins une valve pour le contrôle d'un flux liquide dans le dispositif hydraulique.

23. Appareil selon la revendication 22, dans lequel le dispositif de contrôle (6, 10) comprend un contrôle à distance sans fil pour contrôler la valve.

24. Appareil selon la revendication 21, dans lequel le dispositif de restriction (4, 56) comprend un moyen hydraulique et le dispositif de commande (60) comprend un réservoir (20, 34, 46, 230) formant une chambre de fluide avec un volume variable relié au moyen hydraulique et le dispositif de commande (60) est adapté de façon à distribuer le fluide de la chambre au dispositif hydraulique par réduction du volume de la chambre et à retirer le fluide du moyen hydraulique vers la chambre par expansion du volume de la chambre.

25. Appareil selon la revendication 19 ou 21, dans lequel le dispositif de commande (60) comprend un moteur (16, 18, 36, 232).

26. Appareil selon la revendication 25, dans lequel le moteur (16, 18, 36, 232) comprend un moteur rotatif, et le dispositif de commande (6, 10) contrôle le moteur rotatif de façon à effectuer un nombre de révolutions souhaité.

27. Appareil selon la revendication 25, dans lequel le moteur (16, 18, 36, 232) comprend un moteur linéaire.

28. Appareil selon la revendication 25, dans lequel le moteur (16, 18, 36, 232) comprend un moteur à fluide hydraulique ou pneumatique et le dispositif de contrôle (6, 10) contrôle le moteur à fluide.

29. Appareil selon la revendication 25, dans lequel le moteur (16, 18, 36, 232) comprend un moteur électrique présentant des parties électriquement conductrices constituées de matières plastiques.

30. Appareil selon l'une quelconque des revendications 19 à 21, dans lequel le dispositif de contrôle (6, 10) libère une énergie polarisée à partir de la source d'énergie interne (32, 42, 236).

31. Appareil selon l'une quelconque des revendications 19 à 21, dans lequel le dispositif de contrôle (6, 10) décale la polarité de l'énergie libérée pour inverser le dispositif de commande (60).

32. Appareil selon l'une quelconque des revendications 19 à 21, dans lequel le dispositif de commande (60) comprend un moteur électrique (232) et l'énergie libérée comprend une énergie électrique.

33. Appareil selon l'une quelconque des revendications 19 à 21 et 25, dans lequel le dispositif de restriction (4, 56) peut être commandé pour réaliser une fonction réversible.

34. Appareil selon la revendication 33, comprenant un dispositif d'inversion (14, 38, 52, 234) implantable dans le patient pour inverser la fonction réalisée par le dispositif de restriction (4, 56).

35. Appareil selon la revendication 34, dans lequel le dispositif de contrôle (6, 10) contrôle le dispositif d'inversion (14, 38, 52, 234) pour inverser la fonction réalisée par le dispositif de restriction (4, 56).

36. Appareil selon la revendication 34, dans lequel le dispositif d'inversion (14, 38, 52, 234) comprend un moyen hydraulique comprenant une valve (38) pour décaler la direction du flux d'un fluide dans le moyen hydraulique.

37. Appareil selon la revendication 36, dans lequel le dispositif d'inversion (14, 38, 52, 234) comprend un dispositif d'inversion mécanique (14, 52).

38. Appareil selon la revendication 37, dans lequel le dispositif d'inversion mécanique comprend un interrupteur (14).

39. Appareil selon la revendication 37, dans lequel le dispositif d'inversion (14, 38, 52, 234) comprend une boîte de vitesse (52).

40. Appareil selon la revendication 34, dans lequel le dispositif d'inversion (14, 38, 52, 234) comprend un interrupteur (14).

41. Appareil selon la revendication 40, dans lequel l'interrupteur (14) du dispositif d'inversion peut être commandé par l'énergie libérée.

42. Appareil selon la revendication 41, dans lequel le dispositif de contrôle (6, 10) contrôle la commande de l'interrupteur (14) du dispositif d'inversion par décalage de la polarité de l'énergie libérée fournie à l'interrupteur (14).

43. Appareil selon la revendication 40, dans lequel l'interrupteur (14) comprend un interrupteur électrique et la source d'énergie interne (32, 42, 236) fournit de l'énergie électrique pour la commande de l'interrupteur (14).

44. Appareil selon la revendication 40, dans lequel le dispositif de commande (60) comprend un moteur (16, 18, 36, 232) et un dispositif d'inversion (14, 38, 52, 234) inverse le moteur (16, 18, 36, 232).

45. Appareil selon la revendication 1, 19 à 21, dans lequel le dispositif de restriction (4, 56) comprend un moyen hydraulique et le dispositif de commande (60) est adapté pour conduire un fluide hydraulique dans le moyen hydraulique.

46. Appareil selon la revendication 45, dans lequel le dispositif de commande (60) comprend un moteur (16, 18, 36, 232).

47. Appareil selon la revendication 45 ou 46, dans lequel le dispositif de commande (60) comprend un conduit de fluide relié au moyen hydraulique du dispositif de restriction (4, 56) et un réservoir (20, 34, 46, 230) pour le fluide, le réservoir (20, 34, 46, 230) formant une partie du conduit.

48. Appareil selon la revendication 47, dans lequel le moyen hydraulique et le conduit sont dépourvus de clapet anti-retour.

49. Appareil selon la revendication 48, dans lequel le réservoir (20, 34, 46, 230) forme une chambre de fluide avec un volume variable, et le dispositif de commande (60) est adapté de façon à distribuer le fluide de la chambre du moyen hydraulique du dispositif de restriction (4, 56) par réduction du volume de la chambre et à retirer le fluide du moyen hydraulique vers la chambre par expansion du volume de la chambre.

50. Appareil selon l'une quelconque des revendications précédentes, comprenant en outre au moins un capteur implantable (54) pour capter au moins un paramètre physique du patient.

51. Appareil selon la revendication 50, dans lequel le capteur (54) comprend un capteur de pression pour capter directement ou indirectement comme paramètre physique, la pression dans le côlon (58) ou le rectum.

52. Appareil selon la revendication 50 ou 51, dans lequel le dispositif de contrôle (6, 10) contrôle le dispositif de restriction (4, 56) en réponse aux signaux provenant du capteur (54).

53. Appareil selon la revendication 52, dans lequel la deuxième unité de contrôle (138) contrôle le dispositif de restriction (4, 56) en réponse aux signaux provenant du capteur (54).

54. Appareil selon la revendication 53, dans lequel la première unité de contrôle (136) contrôle le dispositif de restriction (4, 56) en réponse aux signaux provenant du capteur (54).

55. Appareil selon la revendication 54, dans lequel la première unité de contrôle (136) stocke l'information sur le paramètre physique capté par le capteur (54) et est commandée manuellement pour contrôler le dispositif de restriction (4, 56) sur la base des informations stockées.

56. Appareil selon l'une quelconque des revendications 50 à 55, comprenant en outre au moins un émetteur implantable pour émettre l'information sur le paramètre physique capté par le capteur (54).

57. Appareil selon l'une quelconque des revendications précédentes, comprenant en outre un communicateur de données externe destiné à être à l'extérieur du corps du patient et un communicateur de données interne implantable dans le patient pour communiquer avec le communicateur externe, le communicateur de données interne restituant les données concernant le patient, au communicateur de données externe ou le communicateur de données externe fournit des données au communicateur de données interne.

58. Appareil selon la revendication 57, dans lequel le communicateur de données interne fournit des données concernant le dispositif de restriction (4, 56).

59. Appareil selon la revendication 57 ou 58, dans lequel le communicateur implantable fournit des données concernant au moins un signe physique du patient.

60. Appareil selon l'une quelconque des revendications précédentes, dans lequel le dispositif de restriction (4, 56) est adapté de façon à contrôler la voie de passage des matières fécales.

61. Appareil selon la revendication 60, dans lequel le dispositif de restriction (4, 56) peut être commandé pour élargir et restreindre la voie de passage des matières fécales une fois implanté dans le patient.

62. Appareil selon la revendication 60 ou 61, dans lequel le dispositif de restriction (4, 56) est adapté pour contrôler en continu la restriction de la voie de passage des matières fécales, une fois implanté dans le patient.

63. Appareil selon l'une quelconque des revendications précédentes, dans lequel le dispositif de contrôle (6, 10) est adapté de façon à contrôler la source d'énergie externe (10, 40) pour libérer une énergie électrique sans fil et le dispositif de transformation de l'énergie (6, 62) est adapté de façon à transformer l'énergie électrique en énergie cinétique pour commander e dispositif de restriction (4, 56).

64. Appareil selon la revendication 63, dans lequel le dispositif de restriction (4, 56) est commandé directement avec l'énergie cinétique lorsque le dispositif de transformation d'énergie (6, 62) transforme l'énergie électrique en énergie cinétique.

65. Appareil selon l'une quelconque des revendications précédentes, dans lequel le dispositif de contrôle (6, 10) contrôle la source d'énergie interne (32, 42, 236) pour libérer une énergie magnétique, une énergie non magnétique, une énergie électromagnétique, une énergie non électromagnétique, une énergie cinétique, une énergie non cinétique, une énergie sonique, une énergie non sonique, une énergie thermique ou une énergie non thermique.

66. Appareil selon l'une quelconque des revendications précédentes, dans lequel le dispositif de restriction (4, 56) est non gonflable.

67. Appareil selon l'une quelconque des revendications précédentes, dans lequel le dispositif de contrôle (6, 10) contrôle la source d'énergie interne (32, 42, 236) de façon à libérer de l'énergie pendant une durée déterminée.

68. Appareil selon l'une quelconque des revendications 1 à 66, dans lequel le dispositif de contrôle (6, 10) contrôle la source d'énergie externe (10, 40) pour libérer l'énergie en un nombre déterminé d'impulsions d'énergie.

69. Appareil selon l'une quelconque des revendications précédentes, dans lequel le dispositif de contrôle (6, 10) est adapté de façon à contrôler la source d'énergie interne (32, 42, 236) pour libérer de l'énergie de manière non invasive.

70. Appareil selon l'une quelconque des revendications précédentes, dans lequel le dispositif de contrôle (6, 10) comprend un contrôle à distance sans fil pour transmettre au moins un signal de contrôle sans fil pour contrôler le dispositif de restriction (4, 56).

71. Appareil selon la revendication 70, dans lequel le contrôle à distance peut obtenir des informations sur l'état du dispositif de restriction (4, 56) lorsque le dispositif de restriction (4, 56) est implantable et pour contrôler le dispositif de restriction (4, 56) en réponse à l'information.

72. Appareil selon la revendication 70 ou 71, dans lequel le contrôle à distance sans fil comprend au moins un transmetteur ou émetteur-récepteur de signal externe et au moins un récepteur ou émetteur-récepteur de signal interne implantable dans le patient.

73. Appareil selon la revendication 70 ou 71, dans lequel le contrôle à distance sans fil comprend au moins un récepteur ou un émetteur-récepteur de signal externe et au moins un transmetteur ou un émetteur-récepteur de signal interne implantable dans le patient.

74. Appareil selon l'une quelconque des revendications 70 à 73, dans lequel le contrôle à distance est capable d'envoyer des informations concernant le dispositif de restriction (4, 56) à partir de l'intérieur du corps du patient jusqu'à l'extérieur de celui-ci.

75. Appareil selon la revendication 74, dans lequel le contrôle à distance contrôle le dispositif de restriction (4, 56) en réponse à l'information.

76. Appareil selon l'une quelconque des revendications 70 à 75, dans lequel le contrôle à distance transmet un signal porteur pour porter le signal de contrôle.

77. Appareil selon la revendication 76, dans lequel le signal porteur est à modulation de fréquence, à modulation d'amplitude ou à modulation de fréquence et d'amplitude.

78. Appareil selon la revendication 76 ou 77, dans lequel le signal porteur est numérique, analogique ou numérique et analogique.

79. Appareil selon l'une quelconque des revendications 76 à 78, dans lequel le signal de contrôle utilisé avec le signal porteur est à modulation de fréquence, à modulation d'amplitude ou à modulation de fréquence et d'amplitude.

80. Appareil selon l'une quelconque des revendications 70 à 79, dans lequel le signal de contrôle comprend un signal par ondes comprenant un signal quelconque parmi un signal par ondes sonores comprenant un signal par ondes à ultrasons, un signal par ondes électromagnétique comprenant un signal par lumière infrarouge, un signal de lumière visible, un signal de lumière ultraviolette et un signal de lumière laser, un signal par micro-ondes, un signal par ondes radio, un signal par rayonnement de rayons X, un signal par rayonnement gamma.

81. Appareil selon l'une quelconque des revendications 70 à 79 dans lequel le signal de contrôle comprend un champ électrique, magnétique ou électrique et magnétique.

82. Appareil selon l'une quelconque des revendications 70 à 80, dans lequel le signal de contrôle est numérique, analogique ou numérique et analogique.

83. Appareil selon la revendication 82, dans lequel le contrôle à distance transmet un signal par ondes porteuses électromagnétiques pour porter le signal de contrôle numérique ou analogique.

84. Appareil selon l'une quelconque des revendications 70 à 83, dans lequel le signal de contrôle est transmis en impulsions par le contrôle à distance sans fil.

85. Appareil selon l'une quelconque des revendications 14 à 16 ou 61 à 63, comprenant en outre un stabilisateur implantable pour stabiliser l'énergie libérée par le dispositif de contrôle (6, 10).

86. Appareil selon la revendication 85, dans lequel l'énergie libérée par le dispositif de contrôle (6, 10) comprend une énergie électrique et le stabilisateur comprend au moins un condensateur (162).

87. Appareil selon la revendication 1, dans lequel le dispositif de restriction (4, 56) est commandable par l'énergie libérée de manière manuelle, mécanique, interne ou magnétique.

88. Appareil selon la revendication 1, dans lequel le dispositif de restriction (4, 56) est commandable par l'énergie libérée de manière non manuelle, non mécanique, non thermique ou non magnétique.

89. Appareil selon la revendication 14 ou 16, dans lequel le dispositif de contrôle (6, 10) est adapté de façon à contrôler la source d'énergie interne (32, 42, 236) pour libérer l'énergie électrique, et comprend en outre un condensateur implantable pour produire la série d'impulsions d'énergie à partir de l'énergie libérée.

90. Appareil selon la revendication 1, comprenant en outre un dispositif d'ajustement pour ajuster le dispositif de restriction (4, 56) de façon à changer la voie de passage de restriction des matières fécales, le dispositif d'ajustement étant adapté pour ajuster mécaniquement le dispositif de restriction (4, 56) ou adapté pour ajuster de manière hydraulique le dispositif de restriction (4, 56) en utilisant un moyen hydraulique qui est dépourvu de fluide hydraulique de type présentant une viscosité telle qu'elle augmente essentiellement lorsqu'elle est exposée à la chaleur ou à un champ magnétique.

91. Appareil selon la revendication 19, dans lequel le dispositif de commande (60) comprend un dispositif de commande électrique.

92. Appareil selon la revendication 19, dans lequel le dispositif de commande (60) est alimenté par une énergie magnétique, non magnétique, électromagnétique, non électromagnétique, cinétique, non cinétique, thermique ou non thermique.

93. Appareil selon la revendication 1, dans lequel le dispositif de contrôle (6, 10) est activé de manière manuelle ou non manuelle pour contrôler la source d'énergie interne (32, 42, 236) pour libérer de l'énergie.

94. Appareil selon la revendication 1, comprenant en outre des éléments électrique implantables comprenant au moins une sécurité du niveau de tension.

95. Appareil selon la revendication 1, comprenant en outre des éléments électriques implantables comprenant une sécurité du niveau de tension unique.

96. Appareil selon la revendication 94 ou 95, dans lequel les éléments électriques sont dépourvus d'un détecteur de courant et/ou d'un détecteur de niveau de charge.

97. Appareil selon l'une quelconque des revendications 93 à 96, comprenant en outre un condensateur ou un accumulateur implantable, dans lequel la charge ou la décharge du condensateur ou de l'accumulateur est contrôlé par l'utilisation d'une sécurité du niveau de tension.

98. Appareil selon la revendication 14, dans lequel l'énergie libérée comprend une énergie électrique et comprend en outre un condensateur implantable pour produire la série d'impulsions d'énergie.

99. Appareil selon la revendication 97 ou 98, dans lequel le condensateur présente une capacité de moins de 0,1 µF.

100. Appareil selon la revendication 14, dans lequel l'énergie sans fil comprend des ondes électromagnétiques à l'exclusion des ondes radio.

101. Appareil selon la revendication 14 ou 15, dans lequel l'énergie sans fil comprend un signal.

102. Appareil selon la revendication 1, dans lequel le dispositif de transformation de l'énergie implantable (6, 62) est adapté de façon à transformer l'énergie sans fil libérée en énergie différente de l'énergie sans fil pour la commande du dispositif de restriction (4, 56).

103. Appareil selon la revendication 102, dans lequel le dispositif de transformation de l'énergie (6, 62) transforme l'énergie sans fil sous la forme d'ondes sonores en énergie électrique du dispositif de restriction (4, 56).

104. Appareil selon la revendication 103, dans lequel le dispositif de transformation de l'énergie (6, 62) transforme l'énergie sans fil sous la forme d'ondes sonores directement en énergie électrique.

105. Appareil selon la revendication 103 ou 104, dans lequel le dispositif de transformation de l'énergie (6, 62) comprend un condensateur.

106. Appareil selon la revendication 105, dans lequel le condensateur est adapté de façon à produire des impulsions électriques à partir de l'énergie électrique transformée.

107. Appareil selon l'une quelconque des revendications 102 à 106, comprenant en outre un moteur ou une pompe implantable (16, 18, 36, 232) pour commander le dispositif de restriction (4, 56), le moteur ou la pompe (16, 18, 36, 232) étant alimenté par l'énergie transformée.

108. Appareil selon la revendication 1, dans lequel le dispositif de restriction (4, 56) est ajustable de manière non manuelle.

109. Appareil selon l'une quelconque des revendications précédentes, dans lequel le dispositif de restriction (4, 56) est intégré dans un matériau souple ou de type gel.

110. Appareil selon l'une quelconque des revendications précédentes, dans lequel le dispositif de restriction (4,56) est intégré dans un matériau de silicone présentant une dureté inférieure à 20 Shore.

111. Appareil selon la revendication 16, comprenant en outre une source d'énergie activable, implantable dans le patient, dans lequel la source d'énergie activable est activée par une énergie sans fil libérée à partir de la source d'énergie externe (10, 40) pour fournir de l'énergie qui est utilisée en relation avec l'opération du dispositif de restriction (4, 56).

112. Appareil selon la revendication 1, comprenant en outre un interrupteur (238) implantable dans le patient pour commuter directement ou indirectement l'alimentation en énergie à partir d'une source interne d'énergie (32, 42, 236).

113. Appareil selon la revendication 112, dans lequel l'interrupteur (238) affecte directement ou indirectement la fourniture en énergie de la source interne d'énergie (32, 42, 236).

114. Appareil selon la revendication 112, dans lequel l'interrupteur (238) commute entre un mode "arrêt" ("off") dans lequel la source interne d'énergie (32, 42, 236) n'est pas utilisée et un mode "marche" ("on") dans lequel la source d'énergie interne (32, 42, 236) fournit de l'énergie pour la commande du dispositif de restriction (4, 56).

115. Appareil selon la revendication 114, dans lequel l'interrupteur (238) est commandable par l'énergie sans fil libérée à partir de la source d'énergie extérieure (10, 40).

116. Appareil selon la revendication 1, dans lequel le dispositif de contrôle (6, 10) comprend un contrôle à distance sans fil.

117. Appareil selon la revendication 112, dans lequel le dispositif de contrôle (6, 10) comprend un contrôle à distance sans fil pour contrôler la source d'énergie interne (32, 42, 236).

118. Appareil selon la revendication 117, dans lequel l'interrupteur (238) est commandable par l'énergie sans fil à partir de la source d'énergie (10, 40) pour commuter entre un mode "arrêt" ("off") dans lequel la source d'énergie interne (32, 42, 236) et le contrôle à distance ne sont pas utilisés, et un mode "attente" ("standby") dans lequel le contrôle à distance peut contrôler la source d'énergie interne (32, 42, 236) pour fournir de l'énergie pour commander le dispositif de restriction (4, 56).

119. Appareil selon la revendication 113, comprenant en outre une source d'énergie supplémentaire implantable dans le patient pour fournir de l'énergie pour le dispositif de restriction (4, 56), dans lequel l'interrupteur (238) est commandable à partir de la source d'énergie interne (32, 42, 236) pour commuter entre un mode "arrêt" ("off) dans lequel la source d'énergie interne supplémentaire n'est pas utilisée, et un mode "marche" ("on") dans lequel la source d'énergie interne supplémentaire fournit de l'énergie pour la commande du dispositif de restriction (4, 56).

120. Appareil selon la revendication 119, dans lequel le dispositif de contrôle (6, 10) contrôle la source d'énergie interne (32, 42, 236) pour commander l'interrupteur (238).

121. Appareil selon la revendication 120, dans lequel le dispositif de contrôle (6, 10) comprend un contrôle à distance sans fil.

122. Appareil selon la revendication 1, dans lequel la source d'énergie interne (32, 42, 236) comprend une source d'énergie électrique.

123. Appareil selon la revendication 122, dans lequel la source d'énergie électrique comprend au moins un accumulateur, au moins un condensateur ou au moins une batterie rechargeable, ou une combinaison d'au moins un condensateur et au moins une batterie rechargeable.

124. Appareil selon la revendication 122, dans lequel la source d'énergie électrique comprend un accumulateur ou une batterie présentant une durée de vie d'au moins 10 ans.

125. Appareil selon la revendication 1, comprenant en outre un moteur (16, 18, 36, 232) implantable dans le patient pour commander le dispositif de restriction (4, 56) dans lequel le dispositif de contrôle (6, 10) est adapté de façon à contrôler la source d'énergie interne (32, 42, 236) pour alimenter le moteur (16, 18, 36, 232).

126. Appareil selon la revendication 124, dans lequel le moteur (16, 18, 36, 232) comprend un moteur rotatif et le dispositif de contrôle (6, 10) contrôle le moteur rotatif de façon à effectuer un nombre de révolutions souhaité.

127. Appareil selon la revendication 124, dans lequel le moteur (16, 18, 36, 232) comprend un moteur linéaire.

128. Appareil selon la revendication 124, dans lequel le moteur (16, 18, 36, 232) comprend un moteur fluide hydraulique ou pneumatique et le dispositif de contrôle (6, 10) contrôle le moteur à fluide.

129. Appareil selon la revendication 124, dans lequel le moteur (16, 18, 36, 232) comprend un moteur électrique présentant des parties électriques conductrices constituées de matières plastique.

130. Appareil selon la revendication 124, dans lequel le dispositif de contrôle (6, 10) est adapté de façon à contrôler la source d'énergie interne (32, 42, 236) pour alimenter directement le moteur (16, 18, 36, 232) avec l'énergie libérée.

131. Appareil selon la revendication 124, dans lequel le dispositif de contrôle (6, 10) est adapté de façon à contrôler la source d'énergie externe (10, 40) pour libérer l'énergie sans fil sous la forme d'un champ magnétique ou d'ondes électromagnétiques pour alimenter directement le moteur (16, 18, 36, 232) alors que l'énergie sans fil est libérée.

132. Appareil selon la revendication 124, dans lequel la source d'énergie interne (32, 42, 236) comprend au moins un accumulateur, au moins un condensateur ou au moins une batterie rechargeable, ou une combinaison d'au moins un condensateur et d'au moins une batterie rechargeable.

133. Appareil selon la revendication 132, dans lequel la source d'énergie interne (32, 42, 236) comprend une source d'énergie électrique.

134. Appareil selon la revendication 133, dans lequel la source d'énergie électrique comprend un accumulateur ou une batterie présentant une durée de vie d'au moins 10 ans.

135. Appareil selon la revendication 1, comprenant en outre une pompe (16, 18, 36, 232) implantable dans le patient pour commander le dispositif de restriction (4, 56) dans lequel le dispositif de contrôle (6, 10) est adapté de façon à contrôler la source d'énergie interne (32, 42, 236) pour libérer de l'énergie pour commander la pompe (16, 18, 36, 232).

136. Appareil selon la revendication 135, dans lequel le dispositif de contrôle (6, 10) est adapté de façon à contrôler la source d'énergie interne (32, 42, 236) pour commander directement la pompe (16, 18, 36, 232) avec l'énergie libérée.

137. Appareil selon la revendication 1, comprenant en outre une pompe (16, 18, 36, 232) implantable dans le patient pour commander le dispositif de restriction (4, 56) dans lequel le dispositif de contrôle (6, 10) est adapté de façon à contrôler la source d'énergie externe (10, 40) pour libérer de l'énergie sous la forme d'un fluide magnétique ou d'ondes électromagnétiques pour commander directement la pompe (16, 18, 36, 232) lorsque l'énergie sans fil est libérée.

138. Appareil selon la revendication 135 ou 137, dans lequel la pompe (16, 18, 36, 232) n'est pas de type pompe à piston.
